# EUROPEAN PATENT SPECIFICATION

(11) **EP 3 030 676 B1**
(45) Date of publication and mention of the grant of the patent: **10.10.2018**
(21) Application number: 14752914.3
(22) Date of filing: 07.08.2014
(51) Int. Cl.: C12Q 1/68

(54) **PREDICTIVE METHOD FOR BONE FRACTURE RISK IN HORSES**
VERFAHREN ZUR VORHERSAGE DES RISIKOS VON KNOCHENBRÜCHEN IN PFERDEN
MÉTHODE DESTINÉE À PRÉDIRE LE RISQUE DE FRACTURE OSSEUSE CHEZ LE CHEVAL

(30) Priority: 07.08.2013 GB 201314131
(43) Date of publication of application: 15.06.2016
(73) Proprietor: Equine Genetics Research Limited, Newmarket, Suffolk CB8 7UU (GB)
(72) Inventor: BLOTT, Sarah Caroline, Newmarket, Suffolk CB8 7UU (GB); SWINBURNE, June Elizabeth, Newmarket, Suffolk CB8 7UU (GB); VAUDIN, Mark, Newmarket, Suffolk CB8 7UU (GB)
(74) Representative: Gibson, Mark
(86) International application number: PCT/GB2014/052421
(87) International publication number: WO 2015/019097

(56) References cited:
- WO-A2-03/046203
- WO-A2-2010/083294
- TUAN V NGUYEN ET AL: "Genetics and the Individualized Prediction of Fracture", CURRENT OSTEOPOROSIS REPORTS, CURRENT SCIENCE INC, NEW YORK, vol. 10, no. 3, 1 August 2012 (2012-08-01) , pages 236-244, XP035096291, ISSN: 1544-2241, DOI: 10.1007/S11914-012-0113-4
- DRAGAN MILENKOVIC ET AL: "Cytogenetic localization of 136 genes in the horse: comparative mapping with the human genome", MAMMALIAN GENOME, vol. 13, no. 9, 1 September 2002 (2002-09-01), pages 524-534, XP055156828, ISSN: 0938-8990, DOI: 10.1007/s00335-001-2137-4
- MOLLY E. MCCUE ET AL: "A High Density SNP Array for the Domestic Horse and Extant Perissodactyla: Utility for Association Mapping, Genetic Diversity, and Phylogeny Studies", PLOS GENETICS, vol. 8, no. 1, 12 January 2012 (2012-01-12), page e1002451, XP055155449, DOI: 10.1371/journal.pgen.1002451
- KAROL ESTRADA ET AL: "Genome-wide meta-analysis identifies 56 bone mineral density loci and reveals 14 loci associated with risk of fracture", NATURE GENETICS, vol. 44, no. 5, 1 May 2012 (2012-05-01), pages 491-501, XP055078032, ISSN: 1061-4036, DOI: 10.1038/ng.2249
- JESSICA L. PETERSEN ET AL: "Genome-Wide Analysis Reveals Selection for Important Traits in Domestic Horse Breeds", PLOS GENETICS, vol. 9, no. 1, 1 January 2013 (2013-01-01) , page e1003211, XP055058918, ISSN: 1553-7390, DOI: 10.1371/journal.pgen.1003211
- SARAH C BLOTT ET AL: "A genome-wide association study demonstrates significant genetic variation for fracture risk in Thoroughbred racehorses", BMC GENOMICS, BIOMED CENTRAL LTD, LONDON, UK, vol. 15, no. 1, 21 February 2014 (2014-02-21), page 147, XP021180440, ISSN: 1471-2164, DOI: 10.1186/1471-2164-15-147

## Description

### FIELD OF THE INVENTION

The invention relates to a method of predicting fracture risk in a human or animal subject, in particular but not exclusively, predicting fracture risk in a horse.

### BACKGROUND OF THE INVENTION

Metabolic bone disorders are often a cause of bone fragility and increased risk of fracture. A common bone metabolic disorder in humans is osteoporosis; a late-onset disease characterized by low bone mineral density, structural deterioration of bone tissue and an elevated risk of fracture in affected individuals. Bone fragility has an estimated heritability of 16-54% (MacGregor et al. (2000) BMJ 320, 1669-1670; Andrew et al. (2005) J Bone Miner. Res. 20, 67-74; Deng et al. (2000) J Bone Miner. Res. 15, 1243-1252) in humans, depending on fracture site and type, and several genes associated with bone mineral density and fracture risk have been identified in humans and mice (Duncan et al. (2011) PLoS Genetics 7, e1001372; Li et al. (2002) Genomics 79, 734-740), although the genes underlying each of these traits appear to be different (Duncan and Brown (2010) J. Clin. Endocrinol. Metab. 95(6), doi 10.1210/jc.2009-2406; Ralston (2007) Proc. Nutr. Soc. 66, 158-165). Bone fragility has also been reported as an associated phenotype in conditions such as schizophrenia, where affected individuals have been reported to have a 2.5 times higher risk of limb fracture than the general population (Agarwal et al. (2010) European Psychiatric Association (EPA) 18th European Congress of Psychiatry, February 28, 2010. Abstract 374).

Bone fractures with non-traumatic origin occur in thoroughbred racehorses, with the majority of fractures occurring in the distal limbs; bones subject to high-impact and load during exercise and racing. Fracture is the main cause of horse mortality on the racecourse (McKee (1995) Equine Vet. Educ. 7, 202-204), with an average of 60 horses per year suffering a fatal distal limb fracture during racing in the UK (both flat and National Hunt) (Parkin et al. (2004) Equine Vet J. 36, 513-519). The prevalence of all (fatal and non-fatal) fractures occurring during training is about 11% (Verheyen and Wood (2004) Equine Vet J. 36, 167-173). Studies of the pathology of equine fracture indicate some evidence of stress-related damage to the bone prior to fracture, which may be related to metabolic disturbances in bone re-modelling (Stover (2003) Clinical Techniques in Equine Practice 2, 312-322).

There is therefore a pressing need for objective molecular readouts that can predict fracture risk.

### SUMMARY OF THE INVENTION

The scope of the invention is defined by the appended claims. According to a first aspect of the disclosure there is provided a method of predicting fracture risk in a human or animal subject which comprises detecting one or more genetic variations within one or more of the following regions corresponding to the equine genome:
between 13.1 Mb and 15.1 Mb on chromosome 1; and/or
between 51.4 Mb and 54.4 Mb on chromosome 9; and/or
between 61.0 Mb and 67.2 Mb on chromosome 18; and/or
between 55.5 Mb and 57.8 Mb on chromosome 21; and/or
between 38.5 Mb and 39.9 Mb on chromosome 22;
wherein the presence of such genetic variations is indicative of a positive prediction for the likelihood of fracture risk.

### BRIEF DESCRIPTION OF THE FIGURES

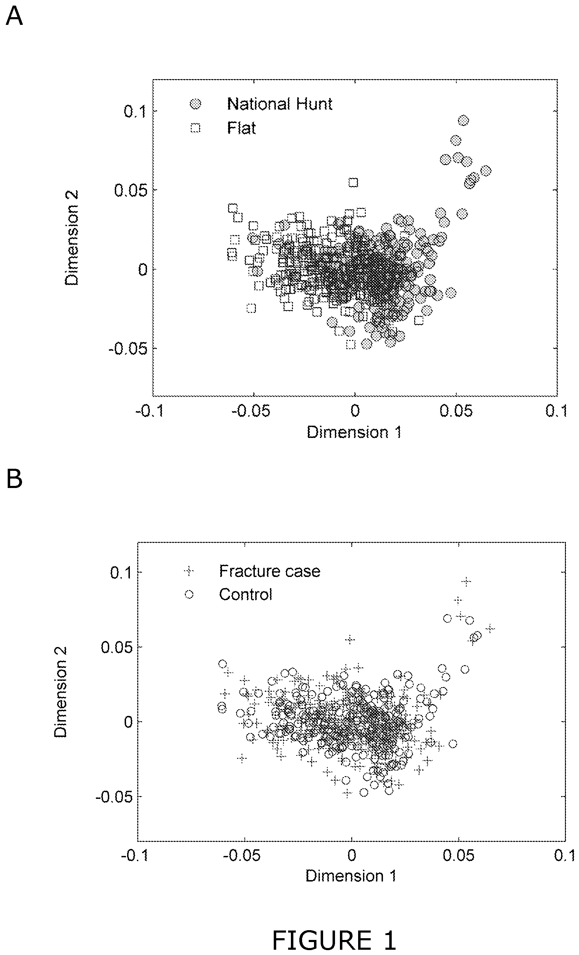

**Figure 1****:** (A) Multidimensional scaling plot showing National Hunt and Flat-bred horses. (B) Multidimensional scaling plot showing fracture cases and controls. Multi-dimensional scaling, based on the identity-by-state (IBS) distance matrix, was used to visualize clustering among individuals. An IBS test showed a significant (p < 2 x 10⁻⁵) difference in IBS between cases and controls, although IBS within groups was not significantly different. There were also significant differences in IBS between flat-bred and National Hunt-bred horses (p < 1 x 10⁻⁵), with evidence for more similarity within groups than between groups (p < 1 x 10⁻⁵, p < 7 x 10⁻⁵).

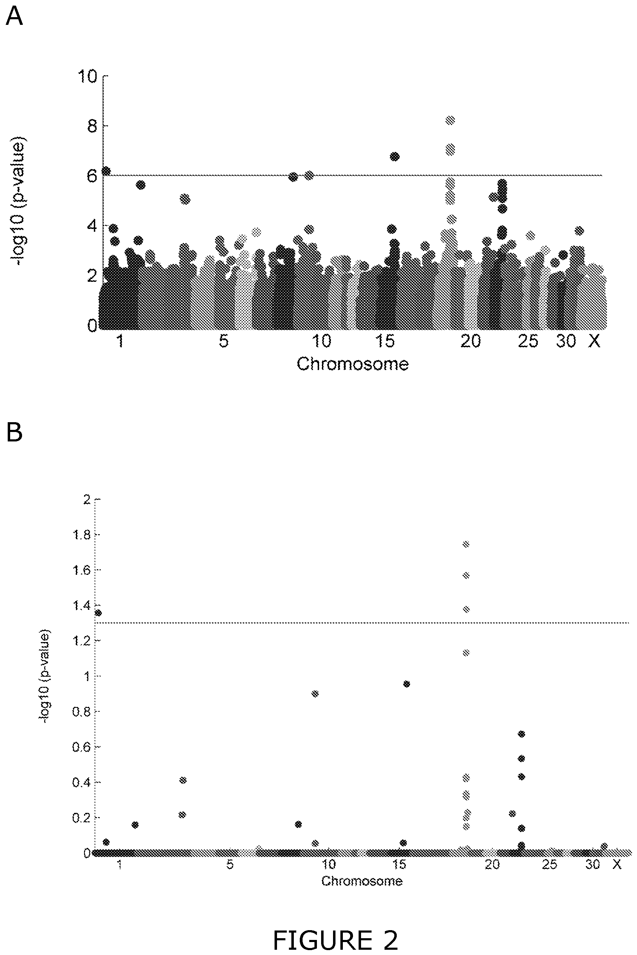

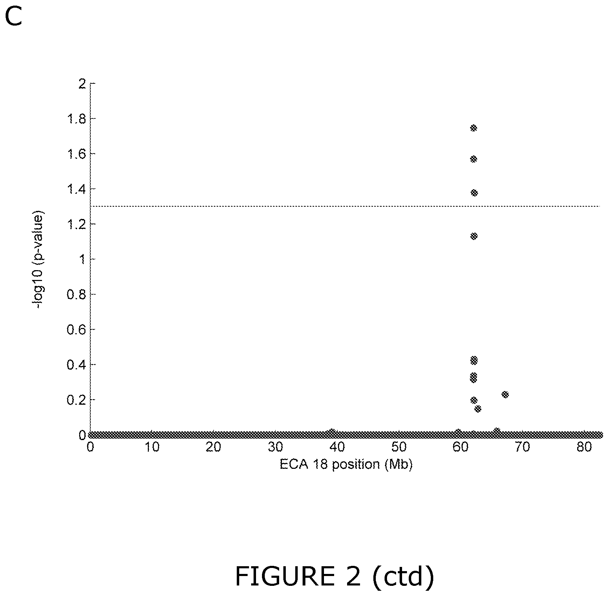

**Figure 2****:** Manhattan plots. (A) Raw p-values from the genome-wide association analysis (Cochran-Mantel-Haenszel test) with Flat and National Hunt-bred horses combined. (B) Empirical p-values calculated after 1000 permutations (C) Empirical p-values for ECA 18 plotted against SNP position on the chromosome (Mb). The 5% genome-wide significance threshold is shown as the horizontal line.

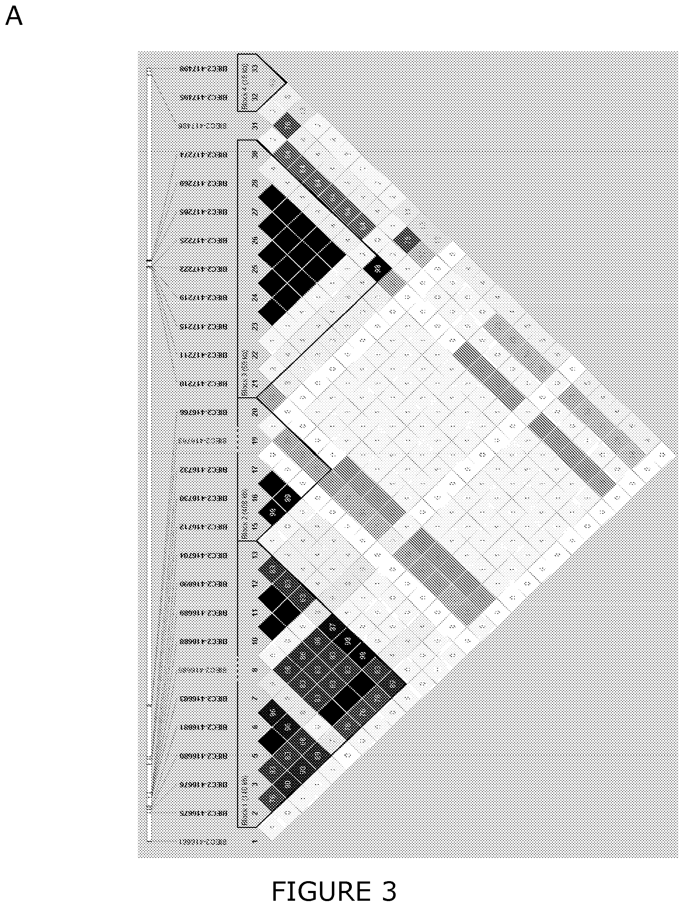

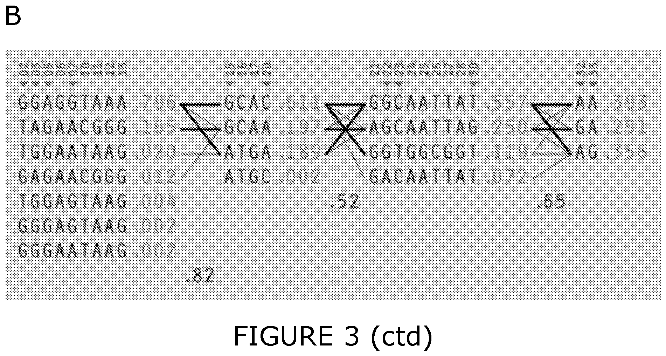

**Figure 3****:** (A) Linkage disequilibrium (LD) around significant SNPs on ECA 18. Haplotype block 1 (62.01 - 62.15 Mb) contains the two most significant SNPs from the genome-wide association study (BIEC2-416680 and BIEC2-416681). There is only one known gene within this haplotype block, *ZNF804A.* Haplotype block 2 (62.15 - 62.76 Mb) contains the candidate gene *FSIP2,* while haplotype block 3 (62.76 - 65.87 Mb) contains the candidate genes *ITGAV, CALCRL, COL3A1* and *COL5A2.* SNP BIEC2-417495 in haplotype block 4 (67.18 - 67.20 Mb) is in linkage disequilibrium (r² = 0.8) with the myostatin (*MSTN*) gene, believed to be associated with racing performance [24, 25, 26], but there is only moderate LD (r² < 0.3) between this SNP and the SNPs in haplotype block 1 which are significantly associated with catastrophic fracture risk. (B) Observed haplotypes and their frequencies for the four haplotype blocks observed in the ECA 18 fracture associated region.

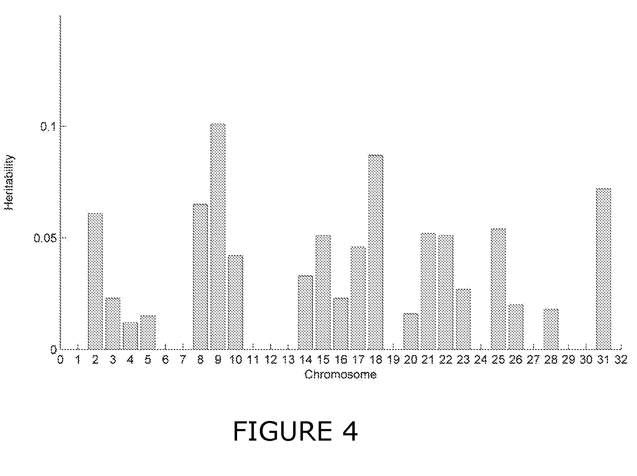

**Figure 4****:** Heritability of fracture risk by chromosome. Estimates of the genetic variance explained by SNPs on individual chromosomes were obtained with Restricted Maximum Likelihood (REML) analysis using the GCTA program.

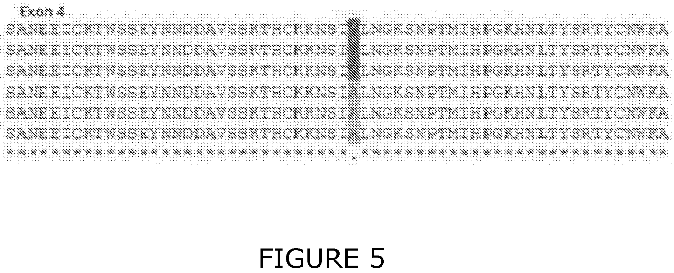

**Figure 5****:** Amino acid sequence showing resulting amino acid change due to SNP identified in ZNF804A at Exon 4 corresponding to 62045643bp of chromosome 18. Sequences from the three control horses are highlighted in dark grey and the three fracture case horses are highlighted in pale grey.

### DETAILED DESCRIPTION OF THE INVENTION

According to a first aspect of the disclosure there is provided a method of predicting fracture risk in a human or animal subject which comprises detecting one or more genetic variations within one or more of the following regions corresponding to the equine genome:
between 13.1 Mb and 15.1 Mb on chromosome 1; and/or
between 51.4 Mb and 54.4 Mb on chromosome 9; and/or
between 61.0 Mb and 67.2 Mb on chromosome 18; and/or
between 55.5 Mb and 57.8 Mb on chromosome 21; and/or
between 38.5 Mb and 39.9 Mb on chromosome 22;
wherein the presence of such genetic variations is indicative of a positive prediction for the likelihood of fracture risk.

Examples of animal subjects include horses, pigs, cattle or dogs. It will be appreciated that the method of the disclosure finds great utility in predicting the susceptibility to injury of a horse, such as a thoroughbred (TB) horse or a racing dog, such as a greyhound. Thus, in one aspect of the disclosure the animal is a horse or a dog. In a further aspect the animal is a horse, such as a thoroughbred (TB) horse. In an alternative aspect the animal is a dog, such as a greyhound.

The predictive method of the invention finds great utility in the detection of healthy horses, such as thoroughbred horses, which are less likely to be susceptible to fracture risk and avoid the potential for distress, and even death, to the animal. There are also significant financial benefits with the predictive method of the invention which will prevent unnecessary funds spent on training the horse when its career is likely to be cut short due to a bone illness likely to result in fracture. A further benefit of the invention is that a positive prediction will allow specific management strategies to be adopted for the individual subject which would minimize the risk of suffering with disease episodes, and more critically prolong the career and life of the individual.

It will be appreciated that in the first instance, the predictive method of the disclosure is able to predict the likelihood of a human or animal subject being at risk or being susceptible to fracture risk. In the second instance, the predictive method of the invention is also able to predict the likelihood of progeny of said human or animal subject being at risk or being susceptible to fracture risk because the method comprises analysis at the genomic level.

It will be appreciated that the genetic variations include any variation in the native or wild type genetic code of the genome from said human or animal subject under analysis. Examples of such genetic variations include: mutations (e.g. point mutations), substitutions, deletions, single nucleotide polymorphisms (SNPs), haplotypes, chromosome abnormalities, Copy Number Variation (CNV), epigenetics and DNA inversions.

References herein to the term "single-nucleotide polymorphism (SNP)" is intended to refer to DNA sequence variation occurring when a single nucleotide in the genome (or other shared sequence) differs between members of a species or between paired chromosomes in an individual.

References herein to the term "haplotype" is intended to refer to a set of genetic markers that are inherited together as a consequence of their chromosomal co-localization. Haplotype may refer to as few as two genetic variants or to an entire chromosome depending on the number of recombination events that have occurred between a given set of variants.

In one embodiment, the one or more genetic variations are within one or more of the following regions corresponding to the equine genome:
between 13.1 Mb and 15.1 Mb on chromosome 1; and/or
between 61.0 Mb and 67.2 Mb on chromosome 18; and/or
between 38.5 Mb and 39.9 Mb on chromosome 22.

In a further embodiment, the one or more genetic variations are within one or more of the following regions corresponding to the equine genome:
between 61.0 Mb and 67.2 Mb on chromosome 18; and/or
between 38.5 Mb and 39.9 Mb on chromosome 22.

In one embodiment, the genetic variation is a SNP present between 13.1 Mb and 14.4 Mb on chromosome 1 and is selected from one or more of the following SNPs: BIEC2-6265, BIEC2-6593-BIEC2-6608, BIEC2-6610-BIEC2-6611, BIEC2-6284, BIEC2-6613-BIEC2-6649, BIEC2-6651-BIEC2-6652, BIEC2-6324, BIEC2-6654-BIEC2-6679, BIEC2-6681-BIEC2-6695, BIEC2-6697, BIEC2-6699-BIEC2-6715, BIEC2-6717, BIEC2-6385, BIEC2-6720-BIEC2-6725, BIEC2-6727-BIEC2-6750, BIEC2-6752-BIEC2-6770, BIEC2-6772-BIEC2-6777, BIEC2-6441, BIEC2-6779-BIEC2-6788, BIEC2-6452, BIEC2-6790-BIEC2-6792, BIEC2-6794-BIEC2-6802, BIEC2-6465, BIEC2-6804-BIEC2-6812, BIEC2-6475-BIEC2-6476, BIEC2-6815-BIEC2-6820, BIEC2-6822-BIEC2-6859, BIEC2-6521, BIEC2-6861-BIEC2-6882, BIEC2-6884-BIEC2-6896, BIEC2-6898-BIEC2-6918, BIEC2-6920-BIEC2-6921, BIEC2-6580, BIEC2-6923-BIEC2-6950, BIEC2-6609, BIEC2-6952-BIEC2-6991, BIEC2-6650, BIEC2-6993-BIEC2-6994, BIEC2-6996, BIEC2-6999, BIEC2-7001-BIEC2-7004, BIEC2-7007-BIEC2-7008, BIEC2-7010-BIEC2-7021, BIEC2-7023-BIEC2-7029, BIEC2-6680, BIEC2-7031-BIEC2-7037, BIEC2-7039-BIEC2-7046, BIEC2-6696, BIEC2-7048, BIEC2-6698, BIEC2-7050-BIEC2-7060, BIEC2-7062-BIEC2-7067, BIEC2-6716, BIEC2-7069-BIEC2-7070, BIEC2-6719, BIEC2-7073, BIEC2-7075-BIEC2-7079, BIEC2-6726, BIEC2-7081-BIEC2-7104, BIEC2-6751, BIEC2-7106-BIEC2-7124, BIEC2-6771, BIEC2-7126-BIEC2-7146, BIEC2-6793, BIEC2-7148, BIEC2-7150-BIEC2-7172, BIEC2-7174-BIEC2-7176, BIEC2-6821, BIEC2-7178-BIEC2-7187, BIEC2-7189-BIEC2-7193, BIEC2-7195-BIEC2-7232, BIEC2-7234-BIEC2-7241, BIEC2-6883, BIEC2-7243-BIEC2-7255, BIEC2-6897, BIEC2-7257-BIEC2-7277, BIEC2-6919, BIEC2-7279-BIEC2-7286, BIEC2-7288, BIEC2-7290-BIEC2-7293, BIEC2-7295-BIEC2-7300 or BIEC2-7302.

In a further embodiment, the genetic variation is a SNP present between 13.1 Mb and 14.4 Mb on chromosome 1 and is selected from one or more of the following SNPs: BIEC2-6265, BIEC2-6284, BIEC2-6324, BIEC2-6385, BIEC2-6441, BIEC2-6452, BIEC2-6465, BIEC2-6475, BIEC2-6476, BIEC2-6521, BIEC2-6580, BIEC2-6609, BIEC2-6650, BIEC2-6680, BIEC2-6696, BIEC2-6698, BIEC2-6716, BIEC2-6719, BIEC2-6726, BIEC2-6751, BIEC2-6771, BIEC2-6793, BIEC2-6821, BIEC2-6883, BIEC2-6897 or BIEC2-6919.

In a further embodiment, the genetic variation is a SNP present between 14.16 and 14.17 Mb on chromosome 1, such as BIEC2-6883.

In one embodiment, the subject is a horse and the genetic variations are within one or more of the following genes: LOC100058684, LOC100066425, LOC100058723, LOC100058765, LOC100067044, PDZD8, LOC100058805, or LOC100058839 located between 13.5 Mb and 15.1 Mb on chromosome 1 of the equine genome.

In one aspect the subject is a human and the genetic variations are within one or more of the following genes: C10orf46, PRLHR, C10orf84, RAB11FIP2, EMX2, PDZD8, SLC18A2 or KCNK18 located between 119 and 120.6 Mb on chromosome 10 of the human genome.

In a further aspect of the disclosure the subject is a human and the genetic variations are within PRLHR located between 120.3 and 120.4 Mb on chromosome 10 of the human genome.

In one embodiment, the genetic variation is a SNP present between 52.3 Mb and 54.4 Mb on chromosome 9 and is selected from one or more of the following SNPs: BIEC2-1094585, BIEC2-1154810-BIEC2-1154822, BIEC2-1154824-BIEC2-1154840, BIEC2-1154842, BIEC2-1154844-BIEC2-1154854, BIEC2-1154856-BIEC2-1154875, BIEC2-1094648-BIEC2-1094649, BIEC2-1154878-BIEC2-1154889, BIEC2-1094662, BIEC2-1154891-BIEC2-1154896, BIEC2-1094669, BIEC2-1154898-BIEC2-1154901, BIEC2-1154903-BIEC2-1154912, BIEC2-1094684, BIEC2-1154914-BIEC2-1154955, BIEC2-1094727, BIEC2-1154958-BIEC2-1154960, BIEC2-1094731, BIEC2-1154962-BIEC2-1154986, BIEC2-1154988-BIEC2-1154991, BIEC2-1094761, BIEC2-1154993-BIEC2-1155009, BIEC2-1094779, BIEC2-1155011-BIEC2-1155016, BIEC2-1094786, BIEC2-1155018-BIEC2-1155028, BIEC2-1094798, BIEC2-1155030-BIEC2-1155042, BIEC2-1094812, BIEC2-1155044-BIEC2-1155045, BIEC2-1094815, BIEC2-1155047-BIEC2-1155053, BIEC2-1094823, BIEC2-1155055-BIEC2-1155067, BIEC2-1094837, BIEC2-1155069-BIEC2-1155142, BIEC2-1094912, BIEC2-1155144-BIEC2-1155147, BIEC2-1094917, BIEC2-1155149-BIEC2-1155150, BIEC2-1094920, BIEC2-1155152, BIEC2-1094922, BIEC2-1094923, BIEC2-1155155-BIEC2-1155161, BIEC2-1094931, BIEC2-1155163, BIEC2-1094933, BIEC2-1155165-BIEC2-1155166, BIEC2-1094936, BIEC2-1155168-BIEC2-1155179, BIEC2-1094949-BIEC2-1094950, BIEC2-1155182-BIEC2-1155191, BIEC2-1094961-BIEC2-1094962, BIEC2-1155194-BIEC2-1155221, BIEC2-1094991, BIEC2-1155223-BIEC2-1155226, BIEC2-1094996, BIEC2-1155228-BIEC2-1155231, BIEC2-1155233-BIEC2-1155261, BIEC2-1155263-BIEC2-1155266, BIEC2-1095034, BIEC2-1155268-BIEC2-1155288, BIEC2-1095056, BIEC2-1155290-BIEC2-1155300, BIEC2-1095068, BIEC2-1155302-BIEC2-1155385, BIEC2-1095153, BIEC2-1155387-BIEC2-1155393, BIEC2-1095161-BIEC2-1095162, BIEC2-1155396-BIEC2-1155457, BIEC2-1095225, BIEC2-1155459-BIEC2-1155464, BIEC2-1155466, BIEC2-1095233-BIEC2-1095234, BIEC2-1155469-BIEC2-1155485, BIEC2-1095252-BIEC2-1095253, BIEC2-1155488-BIEC2-1155495, BIEC2-1095262, BIEC2-1155497-BIEC2-1155513, BIEC2-1095280, BIEC2-1155515-BIEC2-1155528, BIEC2-1095295, BIEC2-1155530-BIEC2-1155547, BIEC2-1095314, BIEC2-1155549-BIEC2-1155556, BIEC2-1095323-BIEC2-1095324, BIEC2-1155559-BIEC2-1155564, BIEC2-1095331, BIEC2-1155566-BIEC2-1155593 or BIEC2-1095359.

In a further embodiment, the genetic variation is a SNP present between 52.3 Mb and 54.4 Mb on chromosome 9 and is selected from one or more of the following SNPs: BIEC2-1094585, BIEC2-1094648, BIEC2-1094649, BIEC2-1094662, BIEC2-1094669, BIEC2-1094684, BIEC2-1094727, BIEC2-1094731, BIEC2-1094761, BIEC2-1094779, BIEC2-1094786, BIEC2-1094798, BIEC2-1094812, BIEC2-1094815, BIEC2-1094823, BIEC2-1094837, BIEC2-1094912, BIEC2-1094917, BIEC2-1094920, BIEC2-1094922, BIEC2-1094923, BIEC2-1094931, BIEC2-1094933, BIEC2-1094936, BIEC2-1094949, BIEC2-1094950, BIEC2-1094961, BIEC2-1094962, BIEC2-1094991, BIEC2-1094996, BIEC2-1095034, BIEC2-1095056, BIEC2-1095068, BIEC2-1095153, BIEC2-1095161, BIEC2-1095162, BIEC2-1095225, BIEC2-1095233, BIEC2-1095234, BIEC2-1095252, BIEC2-1095253, BIEC2-1095262, BIEC2-1095280, BIEC2-1095295, BIEC2-1095314, BIEC2-1095323, BIEC2-1095324, BIEC2-1095331 or BIEC2-1095359.

In a further embodiment, the genetic variation is a SNP present between 53.23 and 53.24 Mb on chromosome 9, such as BIEC2-1094991.

In one embodiment, the subject is a horse and the genetic variations are within one or more of the following genes: OXR1, ABRA, LOC100056482, A8C9U1_HORSE, LOC100063841, LOC100063908, LOC100056570, LOC100056618, NUDCD1, LOC100056701 , LOC100064208, LOC100064267, ENSECAG00000005533, LOC100064295 or LOC100064355 located between 51.4 Mb and 54.4 Mb on chromosome 9 of the equine genome.

In one aspect the subject is a human and the genetic variations are within one or more of the following genes: AC090579.1, ABRA, ANGPT1, RSPO2, EIF3E, TTC35, TMEM74, TRHR, NUDCD1, ENY2, PKHD1L1, EBAG9, SYBU or KCNV1 located between 107 and 111 Mb on chromosome 8 of the human genome.

In one embodiment, the genetic variation is a SNP present between 61.0 Mb and 67.2 Mb on chromosome 18 and is selected from one or more of the following SNPs: BIEC2-438198-BIEC2-438202, BIEC2-416675-BIEC2-416676, BIEC2-438205, BIEC2-416678-BIEC2-416681, BIEC2-438210, BIEC2-416683, BIEC2-438212, BIEC2-416685, BIEC2-438214, BIEC2-416687-BIEC2-416690, BIEC2-438219-BIEC2-438223, BIEC2-438225, BIEC2-438227-BIEC2-438228, BIEC2-438230-BIEC2-438234, BIEC2-416704, BIEC2-438236-BIEC2-438238, BIEC2-416708, BIEC2-438240-BIEC2-438242, BIEC2-416712, BIEC2-438244-BIEC2-438254, BIEC2-438256-BIEC2-438261, BIEC2-416730, BIEC2-438263, BIEC2-416732, BIEC2-438265, BIEC2-438267, BIEC2-416735, BIEC2-438269-BIEC2-438275, BIEC2-416743, BIEC2-438277-BIEC2-438284, BIEC2-438286-BIEC2-438293, BIEC2-438295-BIEC2-438297, BIEC2-416763, BIEC2-438299-BIEC2-438300, BIEC2-416766-BIEC2-416768, BIEC2-438304, BIEC2-416770, BIEC2-438306-BIEC2-438329, BIEC2-438331-BIEC2-438335, BIEC2-416800, BIEC2-438337, BIEC2-416802, BIEC2-438339-BIEC2-438342, BIEC2-416807-BIEC2-416808, BIEC2-438346, BIEC2-438349-BIEC2-438352, BIEC2-416814, BIEC2-438354-BIEC2-438360, BIEC2-416822, BIEC2-438362-BIEC2-438364, BIEC2-416826, BIEC2-438366-BIEC2-438367, BIEC2-438369-BIEC2-438374, BIEC2-438376-BIEC2-438383, BIEC2-416843, BIEC2-438385-BIEC2-438395, BIEC2-416855, BIEC2-438397-BIEC2-438404, BIEC2-438406-BIEC2-438410, BIEC2-416869, BIEC2-438412-BIEC2-438414, BIEC2-416873, BIEC2-438416, BIEC2-416875, BIEC2-438418-BIEC2-438420, BIEC2-416879-BIEC2-416880, BIEC2-438423, BIEC2-416882, BIEC2-438425, BIEC2-438427-BIEC2-438433, BIEC2-416891, BIEC2-438435-BIEC2-438437, BIEC2-416895, BIEC2-438439-BIEC2-438447, BIEC2-416905, BIEC2-438449-BIEC2-438463, BIEC2-416921, BIEC2-438465, BIEC2-416923, BIEC2-438467-BIEC2-438472, BIEC2-438474-BIEC2-438477, BIEC2-416934, BIEC2-438479, BIEC2-416936, BIEC2-438481-BIEC2-438488, BIEC2-416945, BIEC2-438490-BIEC2-438492, BIEC2-416949-BIEC2-416950, BIEC2-438495-BIEC2-438498, BIEC2-416955, BIEC2-438500-BIEC2-438501, BIEC2-438503-BIEC2-438505, BIEC2-438507-BIEC2-438511, BIEC2-416966, BIEC2-438513-BIEC2-438514, BIEC2-416969, BIEC2-438516-BIEC2-438528, BIEC2-416984-BIEC2-416985, BIEC2-438531-BIEC2-438532, BIEC2-416988, BIEC2-438534-BIEC2-438536, BIEC2-416992, BIEC2-438539-BIEC2-438543, BIEC2-416998, BIEC2-438545-BIEC2-438546, BIEC2-417001, BIEC2-438548, BIEC2-417003, BIEC2-438550-BIEC2-438555, BIEC2-417010, BIEC2-438557-BIEC2-438570, BIEC2-438572-BIEC2-438576, BIEC2-417030, BIEC2-438578-BIEC2-438580, BIEC2-417034, BIEC2-438582-BIEC2-438600, BIEC2-417054, BIEC2-438602-BIEC2-438613, BIEC2-417067, BIEC2-438616-BIEC2-438622, BIEC2-417075, BIEC2-438624-BIEC2-438632, BIEC2-417085, BIEC2-438634, BIEC2-417087, BIEC2-438636-BIEC2-438637, BIEC2-417090, BIEC2-438639-BIEC2-438643, BIEC2-417096, BIEC2-438645-BIEC2-438647, BIEC2-417100, BIEC2-438649-BIEC2-438658, BIEC2-417111, BIEC2-438660-BIEC2-438665, BIEC2-417118, BIEC2-438667, BIEC2-417120-BIEC2-417121, BIEC2-438670-BIEC2-438671, BIEC2-417124-BIEC2-417126, BIEC2-438675-BIEC2-438677, BIEC2-417130, BIEC2-438679-BIEC2-438682, BIEC2-417135, BIEC2-438684-BIEC2-438686, BIEC2-417139, BIEC2-438688-BIEC2-438689, BIEC2-417142, BIEC2-438691-BIEC2-438692, BIEC2-417145, BIEC2-438694-BIEC2-438699, BIEC2-417152, BIEC2-438701-BIEC2-438710, BIEC2-417163, BIEC2-438712, BIEC2-417165, BIEC2-438714-BIEC2-438726, BIEC2-417179, BIEC2-438728-BIEC2-438734, BIEC2-417187, BIEC2-438736-BIEC2-438739, BIEC2-417192, BIEC2-438741, BIEC2-417194, BIEC2-438743-BIEC2-438744, BIEC2-438746-BIEC2-438753, BIEC2-438755-BIEC2-438756, BIEC2-417207-BIEC2-417208, BIEC2-438759, BIEC2-417210-BIEC2-417211, BIEC2-438762-BIEC2-438764, BIEC2-417215, BIEC2-438766-BIEC2-438768, BIEC2-417219, BIEC2-438770-BIEC2-438771, BIEC2-417222-BIEC2-417223, BIEC2-438774, BIEC2-417225, BIEC2-438776-BIEC2-438791, BIEC2-438793-BIEC2-438802, BIEC2-438804-BIEC2-438817, BIEC2-417265, BIEC2-438819-BIEC2-438821, BIEC2-417269, BIEC2-438823-BIEC2-438825, BIEC2-417273-BIEC2-417274, BIEC2-438828-BIEC2-438834, BIEC2-417282, BIEC2-438836-BIEC2-438843, BIEC2-417291, BIEC2-438845, BIEC2-417293, BIEC2-438847, BIEC2-417295, BIEC2-438850, BIEC2-438852, BIEC2-417298-BIEC2-417299, BIEC2-438856-BIEC2-438858, BIEC2-417303, BIEC2-438860-BIEC2-438861, BIEC2-417306, BIEC2-438863, BIEC2-417308, BIEC2-438865-BIEC2-438871, BIEC2-438873-BIEC2-438875, BIEC2-417319-BIEC2-417320, BIEC2-438881, BIEC2-438883-BIEC2-438884, BIEC2-417324, BIEC2-438886-BIEC2-438888, BIEC2-417328, BIEC2-438890-BIEC2-438893, BIEC2-417333, BIEC2-438895-BIEC2-438910, BIEC2-417350, BIEC2-438912-BIEC2-438925, BIEC2-417365, BIEC2-438927-BIEC2-438929, BIEC2-417369, BIEC2-438931-BIEC2-438932, BIEC2-417372, BIEC2-438934-BIEC2-438937, BIEC2-438939-BIEC2-438941, BIEC2-438943-BIEC2-438946, BIEC2-417384, BIEC2-438948-BIEC2-438956, BIEC2-438958-BIEC2-438970, BIEC2-417407-BIEC2-417408, BIEC2-438973-BIEC2-438981, BIEC2-417418, BIEC2-438983-BIEC2-438986, BIEC2-417423-BIEC2-417424, BIEC2-438989-BIEC2-439002, BIEC2-439004-BIEC2-439017, BIEC2-417453-BIEC2-417454, BIEC2-439020-BIEC2-439022, BIEC2-417458, BIEC2-439024-BIEC2-439028, BIEC2-417464, BIEC2-439030-BIEC2-439031, BIEC2-417467, BIEC2-439033-BIEC2-439040, BIEC2-417476, BIEC2-439042-BIEC2-439043, BIEC2-417479, BIEC2-439045-BIEC2-439046, BIEC2-417482, BIEC2-439048, BIEC2-417484, BIEC2-439050, BIEC2-417486, BIEC2-439052-BIEC2-439059 or BIEC2-417495.

In a further embodiment, the genetic variation is a SNP present between 61.0 Mb and 67.2 Mb on chromosome 18 and is selected from one or more of the following SNPs: BIEC2-416675, BIEC2-416676, BIEC2-416678, BIEC2-416679, BIEC2-416680, BIEC2-416681, BIEC2-416683, BIEC2-416685, BIEC2-416687, BIEC2-416688, BIEC2-416689, BIEC2-416690, BIEC2-416704, BIEC2-416712, BIEC2-416730, BIEC2-416732, BIEC2-416735, BIEC2-416743, BIEC2-416763, BIEC2-416766, BIEC2-416767, BIEC2-416768, BIEC2-416770, BIEC2-416800, BIEC2-416802, BIEC2-416807, BIEC2-416808, BIEC2-416814, BIEC2-416822, BIEC2-416826, BIEC2-416843, BIEC2-416855, BIEC2-416869, BIEC2-416873, BIEC2-416875, BIEC2-416879, BIEC2-416880, BIEC2-416882, BIEC2-416891, BIEC2-416895, BIEC2-416905, BIEC2-416921, BIEC2-416923, BIEC2-416934, BIEC2-416936, BIEC2-416945, BIEC2-416949, BIEC2-416950, BIEC2-416955, BIEC2-416966, BIEC2-416969, BIEC2-416984, BIEC2-416985, BIEC2-416988, BIEC2-416992, BIEC2-416998, BIEC2-417001, BIEC2-417003, BIEC2-417010, BIEC2-417030, BIEC2-417034, BIEC2-417054, BIEC2-417067, BIEC2-417075, BIEC2-417085, BIEC2-417087, BIEC2-417090, BIEC2-417096, BIEC2-417100, BIEC2-417111, BIEC2-417118, BIEC2-417120, BIEC2-417121, BIEC2-417124, BIEC2-417125, BIEC2-417126, BIEC2-417130, BIEC2-417135, BIEC2-417139, BIEC2-417142, BIEC2-417145, BIEC2-417152, BIEC2-417163, BIEC2-417165, BIEC2-417179, BIEC2-417187, BIEC2-417192, BIEC2-417194, BIEC2-417207, BIEC2-417208, BIEC2-417210, BIEC2-417211, BIEC2-417215, BIEC2-417219, BIEC2-417222, BIEC2-417223, BIEC2-417225, BIEC2-417265, BIEC2-417269, BIEC2-417273, BIEC2-417274, BIEC2-417282, BIEC2-417291, BIEC2-417293, BIEC2-417295, BIEC2-417298, BIEC2-417299, BIEC2-417303, BIEC2-417306, BIEC2-417308, BIEC2-417319, BIEC2-417320, BIEC2-417324, BIEC2-417328, BIEC2-417333, BIEC2-417350, BIEC2-417365, BIEC2-417369, BIEC2-417372, BIEC2-417384, BIEC2-417407, BIEC2-417408, BIEC2-417418, BIEC2-417423, BIEC2-417424, BIEC2-417453, BIEC2-417454, BIEC2-417458, BIEC2-417464, BIEC2-417467, BIEC2-417476, BIEC2-417479, BIEC2-417482, BIEC2-417484, BIEC2-417486 or BIEC2-417495.

In a further embodiment, the genetic variation is a SNP present between 62.0 Mb and 62.8 Mb on chromosome 18, such as BIEC2-438205, BIEC2-416680, BIEC2-416681, BIEC2-438210, BIEC2-416683, BIEC2-438214, BIEC2-438222, BIEC2-438227, BIEC2-416704 or BIEC2-416766.

In a yet further embodiment, the genetic variation is a SNP present between 62.0 Mb and 62.2 Mb on chromosome 18, such as BIEC2-416680, BIEC2-416681 or BIEC2-416704.

In a further embodiment, the genetic variation is a SNP present between 67.1 Mb and 67.2 Mb on chromosome 18, such as BIEC2-417495.

In one embodiment, the subject is a horse and the genetic variations are within one or more of the following genes: ZNF804A, LOC100068553, LOC100068571 LOC100068571, LOC100068634, BOI1HO_HORSE, LOC100054234, LOC100068760, ENSECAG00000003166, LOC100054281, TFPI, ENSECAG00000003186, LOC100054329, ENSECAG00000024768, O97950_HORSE, LOC100054421, ENSECAG00000003220, LOC100069122 or MSTN located between 61.7 Mb and 66.5 Mb on chromosome 18 of the equine genome.

In a further embodiment, the subject is a horse and the genetic variations are within one or more of the following genes: ZNF804A, FSIP2, ITGAV, CALCRL, COL3A1, COL3A2, COL5A2 or MSTN located between 61.7 Mb and 66.5 Mb on chromosome 18 of the equine genome.

In a yet further embodiment, the subject is a horse and the genetic variations are within ZNF804A located between 61.7 Mb and 62.1 Mb on chromosome 18 of the equine genome.

In a still yet further embodiment, the subject is a horse and the genetic variations are within ZNF804A located between 61.7 Mb and 62.1 Mb on chromosome 18 of the equine genome and are selected from one or more of the SNPs listed in Table 1:

**Table 1: Locations and Nature of Mutations Identified in ZNF804A**

| Bp | | | | |
|---|---|---|---|---|
| position on ECA18 | Reference /Control allele | Risk allele | Consequence | Comments |
| 61770400 | T | G | 5' UTR | |
| 61770458 | T | C | 5' UTR | |
| 61982334 | C | G | INTRONIC | |
| 61982620 | G | A | INTRONIC | |
| 61982622 | G | A | INTRONIC | |
| 61983020 | C | T | INTRONIC | 1bp deletion [C] |
| 61983338 | G | A | INTRONIC | |
| 61983518 | C | T | INTRONIC | |
| 61983611 | T | C | INTRONIC | |
| 61983688 | G | C | INTRONIC | |
| 61984166 | G | C | INTRONIC | |
| 61984188 | T | A | INTRONIC | |
| 61984516 | A | C | SYNONYMOUS CODING Exon 2 | |
| 61984560 | A | G | INTRONIC | |
| 61984575 | A | C | INTRONIC | |
| 61984785 | T | C | INTRONIC | |
| 61984913 | C | T | INTRONIC | |
| 61984955 | G | A | INTRONIC | |
| 61985069 | G | A | INTRONIC | |
| 61985156 | G | A | INTRONIC | |
| 61985268 | C | T | INTRONIC | T insertion in T rich area but only affects cases |
| 61985468 | C | G | INTRONIC | |
| 61985689 | C | T | INTRONIC | |
| 61985847 | A | G | INTRONIC | |
| 61985973 | G | A | INTRONIC | |
| 61986362 | G | C | INTRONIC | |
| 61986492 | C | T | INTRONIC | |
| 61988314 | C | G | INTRONIC | |
| 61988359 | G | C | INTRONIC | |
| 61989823 | A | G | INTRONIC | |
| 61990225 | T | C | INTRONIC | |
| 61991284 | T | A | INTRONIC | Low read depth, almost appears to be swapped with following base |
| 61991285 | A | T | INTRONIC | Low read depth, almost appears to be swapped with preceding base |
| 61991896 | T | G | INTRONIC | |
| 61992691 | A | T | INTRONIC | |
| 61993470 | G | A | INTRONIC | |
| 61994879 | T | C | INTRONIC | |
| 61995778 | C | A | INTRONIC | |
| 61995793 | A | T | INTRONIC | At start of short run of Ts but good read depth and only present in cases |
| 61996457 | T | C | INTRONIC | |
| 61996958 | C | G | INTRONIC | |
| 61997603 | T | C | INTRONIC | |
| 61997644 | T | G | INTRONIC | |
| 61998009 | A | G | INTRONIC | |
| 61998487 | T | C | INTRONIC | |
| 61998488 | G | A | INTRONIC | |
| 61999113 | C | A | INTRONIC | |
| 61999218 | G | A | INTRONIC | |
| 62000023 | | INS | INTRONIC | T insertion at start of run of Ts but only present in cases |
| 62001789 | A | G | INTRONIC | |
| 62002255 | T | C | INTRONIC | |
| 62002340 | T | C | INTRONIC | Low read depth |
| 62004585 | T | G | INTRONIC | |
| 62005522 | A | C | INTRONIC | |
| 62006515 | G | A | INTRONIC | |
| 62006619 | T | C | INTRONIC | |
| 62007964 | A | C | INTRONIC | |
| 62008083 | G | T | INTRONIC | Known SNP is G to T |
| 62009037 | C | T | INTRONIC | |
| 62010203 | C | G | INTRONIC | |
| 62010516 | A | C | INTRONIC | |
| 62010825 | | DEL | INTRONIC | 5bp deletion [ATAAG] |
| 62014088 | T | C | INTRONIC | |
| 62014970 | | DEL | INTRONIC | T deletion of one or two base pairs at start of run of Ts - present in high numbers in cases but also in some reads in controls |
| 62015422 | A | G | INTRONIC | |
| 62016454 | C | T | INTRONIC | |
| 62017626 | G | T | INTRONIC | |
| 62017921 | A | G | INTRONIC | |
| 62017949 | T | C | INTRONIC | |
| 62019362 | A | G | INTRONIC | |
| 62019972 | A | T | INTRONIC | |
| 62021258 | A | G | INTRONIC | |
| 62021968 | C | A | INTRONIC | |
| 62022204 | A | G | INTRONIC | |
| 62022208 | G | A | INTRONIC | |
| 62022253 | T | G | INTRONIC | |
| 62023683 | | INS | INTRONIC | AT insertion |
| 62023723 | T | G | INTRONIC | |
| 62023961 | G | A | INTRONIC | |
| 62026277 | G | A | INTRONIC | |
| 62027101 | C | T | INTRONIC | |
| 62027818 | T | A | INTRONIC | |
| 62030002 | A | T | INTRONIC | Low read depth |
| 62032182 | G | C | INTRONIC | |
| 62032797 | | DEL | INTRONIC | Low read depth, single T deletion at start of run of Ts |
| 62032876 | A | G | INTRONIC | |
| 62034335 | G | A | INTRONIC | |
| 62035484 | A | G | INTRONIC | Listed as false SNP, found in Icelandics |
| 62035738 | T | G | INTRONIC | |
| 62038431 | G | A | INTRONIC | |
| 62038995 | T | C | INTRONIC | |
| 62041190 | T | A | INTRONIC | |
| 62042238 | A | G | INTRONIC | |
| 62042281 | A | G | INTRONIC | |
| 62042463 | T | C | INTRONIC | |
| 62043711 | C | A | INTRONIC | |
| 62045643 | T | C | NON-SYNONYMOUS CODING Exon 4 | |
| 62047178 | T | A | 3' UTR | |
| 62047184 | A | G | 3' UTR | |
| 62047262 | A | G | 3' UTR | |
| 62047293 | C | T | 3' UTR | |
| 62047491 | | INS | 3' UTR | T insertion at start of run of Ts |
| 62047814 | G | A | DOWNSTREAM | |
| 62047905 | A | C | DOWNSTREAM | |
| 62048027 | A | G | DOWNSTREAM | |
| 62048096 | C | T | DOWNSTREAM | |
| 62048154 | A | G | DOWNSTREAM | All cases between 25 & 30% G with remainder A, low read depth |
| 62048155 | | DEL | DOWNSTREAM | 3bp deletion [AAA] |
| 62048159 | A | T | DOWNSTREAM | All cases between 25 & 30% T, remainder A. Low read depth. |

Of all the mutations identified herein within ZNF804A, only 2 SNPs were present in exons. Of these only one resulted in a change to the amino acid sequence. Located in exon 4, it resulted in the replacement of a T in the control DNA with a C in the case DNA, leading to a change from Valine to Alanine in the amino acid sequence. These changes are displayed in Figure 5. Thus, in a yet further embodiment, the genetic variation is selected from the SNP within Exon 4 of ZNF804A at base pair position 62045643 of chromosome 18.

In a yet further embodiment, the genetic variation is selected from the 3'-UTR of ZNF804A, such as those listed in Table 1 at base pair positions 62047178, 62047184, 62047262 and 62047293 of chromosome 18.

In an alternative embodiment, the genetic variation is selected from the 5'-UTR of ZNF804A, such as those listed in Table 1 at base pair positions 61770400 and 61770458 of chromosome 18.

In an alternative embodiment, the genetic variation is selected from the intronic region of ZNF804A, such as those listed in Table 1.

In an alternative embodiment, the genetic variation is selected from the downstream region of ZNF804A, such as those listed in Table 1 at base pair positions 62047814, 62047905, 62048027, 62048096, 62048154, 62048155 and 62048159 of chromosome 18.

In one embodiment, the subject is a horse and the genetic variations are within MSTN located between 61.7 Mb and 66.5 Mb on chromosome 18 of the equine genome. In a further embodiment, the genetic variation within MSTN is at base pair position 66493737 of chromosome 18.

In one aspect of the disclosure the subject is a human and the genetic variations are within one or more of the following genes: ZNF804A, FSIP2, ZC3H15, ITGAV, FAM171B, ZSWIM2, CALCRL, TFPI, GULP1, COL3A1, COL3A2, COL5A2,WDR75 or MSTN located between 185.4 and 190.93 Mb on chromosome 2 of the human genome.

In a further aspect the subject is a human and the genetic variations are within one or more of the following genes: ZNF804A, FSIP2, ITGAV, CALCRL, COL3A1, COL3A2, COL5A2 or MSTN located between 185.4 and 190.93 Mb on chromosome 2 of the human genome.

In a yet further aspect the subject is a human and the genetic variations are within ZNF804A located between 185.4 and 185.6 Mb on chromosome 2 of the human genome.

In one embodiment, the genetic variation is a SNP present between 56.0 Mb and 57.8 Mb on chromosome 21 and is selected from one or more of the following SNPs: BIEC2-573911, BIEC2-603105-BIEC2-603109, BIEC2-573917, BIEC2-603111-BIEC2-603113, BIEC2-573921, BIEC2-603115-BIEC2-603116, BIEC2-573924, BIEC2-603118-BIEC2-603121, BIEC2-573929-BIEC2-573930, BIEC2-603124-BIEC2-603127, BIEC2-573935-BIEC2-573936, BIEC2-603130-BIEC2-603137, BIEC2-573945, BIEC2-603139-BIEC2-603145, BIEC2-573953, BIEC2-603147-BIEC2-603165, BIEC2-573973, BIEC2-603167-BIEC2-603175, BIEC2-573983, BIEC2-603177-BIEC2-603187, BIEC2-573995, BIEC2-603189, BIEC2-573997, BIEC2-603191-BIEC2-603200, BIEC2-574008, BIEC2-603202, BIEC2-574010, BIEC2-603204-BIEC2-603211, BIEC2-574019, BIEC2-603213-BIEC2-603223, BIEC2-574031, BIEC2-603225, BIEC2-574033, BIEC2-603227-BIEC2-603228, BIEC2-574036, BIEC2-603230-BIEC2-603242, BIEC2-574050, BIEC2-603244-BIEC2-603256, BIEC2-574064, BIEC2-603258-BIEC2-603259, BIEC2-574067, BIEC2-603261-BIEC2-603269, BIEC2-574077, BIEC2-603271-BIEC2-603273, BIEC2-574081, BIEC2-603275-BIEC2-603276, BIEC2-574084, BIEC2-603278, BIEC2-574086-BIEC2-574089, BIEC2-603283-BIEC2-603284, BIEC2-574092-BIEC2-574093, BIEC2-603287-BIEC2-603295, BIEC2-574103, BIEC2-603297-BIEC2-603300, BIEC2-574108-BIEC2-574109, BIEC2-603303, BIEC2-574111, BIEC2-603305, BIEC2-574113-BIEC2-574114, BIEC2-603308, BIEC2-574116, BIEC2-603310-BIEC2-603321, BIEC2-574129-BIEC2-574130 or BIEC2-603324-BIEC2-603326.

In a further embodiment, the genetic variation is a SNP present between 56.0 Mb and 57.8 Mb on chromosome 21 and is selected from one or more of the following SNPs: BIEC2-573911, BIEC2-573917, BIEC2-573921, BIEC2-573924, BIEC2-573929, BIEC2-573930, BIEC2-573935, BIEC2-573945, BIEC2-573953, BIEC2-573973, BIEC2-573983, BIEC2-573995, BIEC2-573997, BIEC2-574008, BIEC2-574010, BIEC2-574019, BIEC2-574031, BIEC2-574033, BIEC2-574036, BIEC2-574050, BIEC2-574064, BIEC2-574067, BIEC2-574077, BIEC2-574081, BIEC2-574084, BIEC2-574086, BIEC2-574087, BIEC2-574088, BIEC2-574089, BIEC2-574092, BIEC2-574093, BIEC2-574103, BIEC2-574108, BIEC2-574109, BIEC2-574111, BIEC2-574113, BIEC2-574114, BIEC2-574116, BIEC2-574129 or BIEC2-574130.

In a further embodiment, the genetic variation is a SNP present between 57.03 and 57.04 Mb on chromosome 21, such as BIEC2-574084.

In one embodiment, the subject is a horse and the genetic variations are within one or more of the following genes: LOC100071850, LOC100147154 , LOC100071879, LOC100071885, LOC100071898, SLC6A3, CLPTM1L, TERT, LOC100058040 or LOC100057994 located between 55.5 Mb and 57 Mb on chromosome 21 of the equine genome.

In one aspect of the disclosure the subject is a human and the genetic variations are within one or more of the following genes: IRX2, IRX4, NDUFS6, MRPL36, LPCAT1, SLC6A3, CLPTM1L, TERT, SLC12A7 or NKD2 located between 1 Mb and 2.7 Mb on chromosome 5 of the human genome.

In one embodiment, the genetic variation is a SNP present between 38.5 Mb and 39.9 Mb on chromosome 22 and is selected from one or more of the following SNPs: BIEC2-626347, BIEC2-626349, BIEC2-626351, BIEC2-626353, BIEC2-595964, BIEC2-626355-BIEC2-626358, BIEC2-595969, BIEC2-626361-BIEC2-626362, BIEC2-595972, BIEC2-626364-BIEC2-626376, BIEC2-595986, BIEC2-626378-BIEC2-626389, BIEC2-626391-BIEC2-626406, BIEC2-596015, BIEC2-626408-BIEC2-626409, BIEC2-626411-BIEC2-626449, BIEC2-626451-BIEC2-626472, BIEC2-596079, BIEC2-626474-BIEC2-626500, BIEC2-626502-BIEC2-626511, BIEC2-626513-BIEC2-626514, BIEC2-596119, BIEC2-626516-BIEC2-626530, BIEC2-596135-BIEC2-596136, BIEC2-626534-BIEC2-626545, BIEC2-626548-BIEC2-626559-BIEC2-626563, BIEC2-626565-BIEC2-626566, BIEC2-626568-BIEC2-626576, BIEC2-596175, BIEC2-626578-BIEC2-626591, BIEC2-626593-BIEC2-626594, BIEC2-596192-BIEC2-596193, BIEC2-626597-BIEC2-626603, BIEC2-626605, BIEC2-626607-BIEC2-626625, BIEC2-626627-BIEC2-626632, BIEC2-626634-BIEC2-626654, BIEC2-626656, BIEC2-626658-BIEC2-626660, BIEC2-626662-BIEC2-626668, BIEC2-596259, BIEC2-626672-BIEC2-626678, BIEC2-626681-BIEC2-626684, BIEC2-626686-BIEC2-626690, BIEC2-596276, BIEC2-626692-BIEC2-626704, BIEC2-626706-BIEC2-626721, BIEC2-626723-BIEC2-626727, BIEC2-596311, BIEC2-626729-BIEC2-626736, BIEC2-626738-BIEC2-626740, BIEC2-626742-BIEC2-626745, BIEC2-626747-BIEC2-626758, BIEC2-626760-BIEC2-626764, BIEC2-626766, BIEC2-626769-BIEC2-626772, BIEC2-626775-BIEC2-626776, BIEC2-626778-BIEC2-626787, BIEC2-626789-BIEC2-626792, BIEC2-626797, BIEC2-626799-BIEC2-626805, BIEC2-596376, BIEC2-626808-BIEC2-626811, BIEC2-626813-BIEC2-626814, BIEC2-626816-BIEC2-626818, BIEC2-626820-BIEC2-626939, BIEC2-596506, BIEC2-626941-BIEC2-626944, BIEC2-596511, BIEC2-626946-BIEC2-626951, BIEC2-596518-BIEC2-596519, BIEC2-626954-BIEC2-626963, BIEC2-596530, BIEC2-626965-BIEC2-626975, BIEC2-596542, BIEC2-626977-BIEC2-626979, BIEC2-596546, BIEC2-626981-BIEC2-626996, BIEC2-626998-BIEC2-627004, BIEC2-627006-BIEC2-627032, BIEC2-627034-BIEC2-627046, BIEC2-596610, BIEC2-627048-BIEC2-627064, BIEC2-596628, BIEC2-627066-BIEC2-627071, BIEC2-627073-BIEC2-627078, BIEC2-627080-BIEC2-627087, BIEC2-627089-BIEC2-627102, BIEC2-627104, BIEC2-627106-BIEC2-627110, BIEC2-627112-BIEC2-627126, BIEC2-627128, BIEC2-627130-BIEC2-627133, BIEC2-627135-BIEC2-627137, BIEC2-627139-BIEC2-627140, BIEC2-596694, BIEC2-627142, BIEC2-627144-BIEC2-627146, BIEC2-627149-BIEC2-627156, BIEC2-627158-BIEC2-627161, BIEC2-627163-BIEC2-627189, BIEC2-627192-BIEC2-627200, BIEC2-596747, BIEC2-627202-BIEC2-627206, BIEC2-627208-BIEC2-627228, BIEC2-627230-BIEC2-627245, BIEC2-627247-BIEC2-627248, BIEC2-627250-BIEC2-627252, BIEC2-627254-BIEC2-627263, BIEC2-596805, BIEC2-627265-BIEC2-627280, BIEC2-627282-BIEC2-627285, BIEC2-627287, BIEC2-627289-BIEC2-627291, BIEC2-596830, BIEC2-627294-BIEC2-627310, BIEC2-627312-BIEC2-627341, BIEC2-627344-BIEC2-627349, BIEC2-596884, BIEC2-627351-BIEC2-627352, BIEC2-627354-BIEC2-627373, BIEC2-627375, BIEC2-627377-BIEC2-627401, BIEC2-596933, BIEC2-627403-BIEC2-627423, BIEC2-596955, BIEC2-627425-BIEC2-627460, BIEC2-596992, BIEC2-627463-BIEC2-627475, BIEC2-627477, BIEC2-627479-BIEC2-627527, BIEC2-627529-BIEC2-627537, BIEC2-627540-BIEC2-627553, BIEC2-627555-BIEC2-627556, BIEC2-597081, BIEC2-627558-BIEC2-627565, BIEC2-627568-BIEC2-627572, BIEC2-597095, BIEC2-627574-BIEC2-627579, BIEC2-627581-BIEC2-627583, BIEC2-597105, BIEC2-627585-BIEC2-627605, BIEC2-627607-BIEC2-627611, BIEC2-627614-BIEC2-627616, BIEC2-627618-BIEC2-627628, BIEC2-597146, BIEC2-627630-BIEC2-627653 or BIEC2-627655-BIEC2-627663.

In a further embodiment, the genetic variation is a SNP present between 38.5 Mb and 39.9 Mb on chromosome 22 and is selected from one or more of the following SNPs: BIEC2-595964, BIEC2-595969, BIEC2-595972, BIEC2-595986, BIEC2-596015, BIEC2-596079, BIEC2-596119, BIEC2-596135, BIEC2-596136, BIEC2-596175, BIEC2-596192, BIEC2-596193, BIEC2-596259, BIEC2-596276, BIEC2-596311, BIEC2-596376, BIEC2-596506, BIEC2-596511, BIEC2-596518, BIEC2-596519, BIEC2-596530, BIEC2-596542, BIEC2-596546, BIEC2-596610, BIEC2-596628, BIEC2-596694, BIEC2-596747, BIEC2-596805, BIEC2-596830, BIEC2-596884, BIEC2-596933, BIEC2-596955, BIEC2-596992, BIEC2-597081, BIEC2-597095, BIEC2-597105 or BIEC2-597146.

In a further embodiment, the genetic variation is a SNP present between 38.5 Mb and 39.18 Mb on chromosome 22, such as BIEC2-595969, BIEC2-596079, BIEC2-596530, BIEC2-596542 or BIEC2-596546.

In one embodiment, the subject is a horse and the genetic variations are within one or more of the following genes: LOC100052066, LOC100049830, LOC100052187, LOC100049902, LOC100052367, LOC100052426, LOC100052484, LOC100052657, LOC100052707, SALL4 or ZFP64 located between 38.5 Mb and 39.9 Mb on chromosome 22 of the equine genome.

In a further embodiment, the subject is a horse and the genetic variations are within ZFP64 located between 39.7 Mb and 39.9 Mb on chromosome 22 of the equine genome.

In a further aspect of the disclosure the subject is a human and the genetic variations are within one or more of the following genes: PTPN1, FAM65C , PARD6B, ADNP, DPM1, MOCS3, KCNG1, NFATC2, ATP9A, SALL4 or ZFP64 located between 49.1 Mb and 50.9 Mb on chromosome 20 of the human genome.

In one aspect the subject is a human and the genetic variations are within one or more of the following genes: KCNG1, NFATC2 or ZFP64 located between 49.1 Mb and 50.9 Mb on chromosome 20 of the human genome.

The methods of the disclosure may be used to detect genetic variations using a biological sample obtained from the human or animal subject. Thus, in one embodiment, the method initially comprises the step of obtaining a biological sample from the human or animal subject.

The nucleic acid may be isolated from the sample according to any methods well known to those of skill in the art. Examples include tissue samples or any cell-containing or acellular biomaterial (i.e. a bodily fluid or hair sample). Biological samples may be obtained by standard procedures and may be used immediately or stored, under conditions appropriate for the type of biological sample, for later use.

Methods of obtaining biological samples are well known to those of skill in the art and include, but are not limited to, aspirations, tissue sections, drawing of blood or other fluids, surgical or needle biopsies, and the like. Examples of suitable biological samples include: whole blood, blood serum, plasma, urine, saliva, or other bodily fluid (stool, tear fluid, synovial fluid, sputum), hair, cerebrospinal fluid (CSF), or an extract or purification therefrom, or dilution thereof. Biological samples also include tissue homogenates, tissue sections and biopsy specimens from a live subject. The samples can be prepared, for example where appropriate diluted or concentrated, and stored in the usual manner.

If necessary, the sample may be collected or concentrated by centrifugation and the like. The cells of the sample may be subjected to lysis, such as by treatments with enzymes, heat, surfactants, ultrasonication, or a combination thereof. The lysis treatment is performed in order to obtain a sufficient amount of nucleic acid derived from the cells of the human or animal subject to detect using polymerase chain reaction.

Methods of plasma and serum preparation are well known in the art. Either "fresh" blood plasma or serum, or frozen (stored) and subsequently thawed plasma or serum may be used. Frozen (stored) plasma or serum should optimally be maintained at storage conditions of -20 to -70°C until thawed and used. "Fresh" plasma or serum should be refrigerated or maintained on ice until used, with nucleic acid (e.g., RNA, DNA or total nucleic acid) extraction being performed as soon as possible. Exemplary methods are described below.

Blood can be drawn by standard methods into a collection tube, typically siliconized glass, either without anticoagulant for preparation of serum, or with EDTA, sodium citrate, heparin, or similar anticoagulants for preparation of plasma. If preparing plasma or serum for storage, although not an absolute requirement, is that plasma or serum is first fractionated from whole blood prior to being frozen. This reduces the burden of extraneous intracellular RNA released from lysis of frozen and thawed cells which might reduce the sensitivity of the amplification assay or interfere with the amplification assay through release of inhibitors to PCR such as porphyrins and hematin. "Fresh" plasma or serum may be fractionated from whole blood by centrifugation, using gentle centrifugation at 300-800 times gravity for five to ten minutes, or fractionated by other standard methods. High centrifugation rates capable of fractionating out apoptotic bodies should be avoided.

It will be appreciated that the step of detecting the one or more genetic variations within one or more of the chromosome regions defined herein will comprise any suitable technique for genetic analysis.

The volume of plasma or serum used in the extraction may vary, but volumes of 1 to 100 ml of plasma or serum are usually sufficient.

Various methods of extraction are suitable for isolating the nucleic acid. Suitable methods include phenol and chloroform extraction. See Maniatis et al., Molecular Cloning, A Laboratory Manual, 2d, Cold Spring Harbor Laboratory Press, page 16.54 (1989).

Numerous commercial kits also yield suitable DNA and RNA including, but not limited to, QIAamp™ mini blood kit, Agencourt Genfind™, Roche Cobas® Roche MagNA Pure® or phenol: chloroform extraction using Eppendorf Phase Lock Gels®, and the NucliSens extraction kit (Biomerieux, Marcy I'Etoile, France). In other methods, mRNA may be extracted from blood/bone marrow samples using MagNA Pure LC mRNA HS kit and Mag NA Pure LC Instrument (Roche Diagnostics Corporation, Roche Applied Science, Indianapolis, IN).

Nucleic acid extracted from tissues, cells, plasma, serum or hair can be amplified using nucleic acid amplification techniques well known in the art. Many of these amplification methods can also be used to detect the presence of genetic variations simply by designing oligonucleotide primers or probes to interact with or hybridize to a particular target sequence in a specific manner. By way of example, but not by way of limitation, these techniques can include the polymerase chain reaction (PCR), reverse transcriptase polymerase chain reaction (RT-PCR), nested PCR, ligase chain reaction. See Abravaya, K., et al, Nucleic Acids Research, 23:675-682, (1995), branched DNA signal amplification, Urdea, M. S., et al, AIDS, 7 (suppl 2):S11-S 14, (1993), amplifiable RNA reporters, Q-beta replication, transcription-based amplification, boomerang DNA amplification, strand displacement activation, cycling probe technology, isothermal nucleic acid sequence based amplification (NASBA). See Kievits, T. et al, J Virological Methods, 35:273-286, (1991), Invader Technology, or other sequence replication assays or signal amplification assays. These methods of amplification each described briefly below and are well-known in the art.

Some methods employ reverse transcription of RNA to cDNA. Various reverse transcriptases may be used, including, but not limited to, MMLV RT, RNase H mutants of MMLV RT such as Superscript and Superscript II (Life Technologies, GIBCO BRL, Gaithersburg, Md.), AMV RT, and thermostable reverse transcriptase from Thermus Thermophilus. For example, one method, but not the only method, which may be used to convert RNA extracted from plasma or serum to cDNA is the protocol adapted from the Superscript II Preamplification system (Life Technologies, GIBCO BRL, Gaithersburg, Md.; catalog no. 18089-011), as described by Rashtchian, A., PCR Methods Applic, 4:S83-S91, (1994).

A variety of amplification enzymes are well known in the art and include, for example, DNA polymerase, RNA polymerase, reverse transcriptase, Q-beta replicase, thermostable DNA and RNA polymerases. Because these and other amplification reactions are catalyzed by enzymes, in a single step assay the nucleic acid releasing reagents and the detection reagents should not be potential inhibitors of amplification enzymes if the ultimate detection is to be amplification based. Amplification methods suitable for use with the present methods include, for example, strand displacement amplification, rolling circle amplification, primer extension preamplification, or degenerate oligonucleotide PCR (DOP).

In one embodiment, PCR is used to amplify a target or marker sequence of interest. The person skilled in the art is capable of designing and preparing primers that are appropriate for amplifying a target sequence. The length of the amplification primers depends on several factors including the nucleotide sequence identity and the temperature at which these nucleic acids are hybridized or used during *in vitro* nucleic acid amplification. The considerations necessary to determine a preferred length for an amplification primer of a particular sequence identity are well-known to the person skilled in the art. For example, the length of a short nucleic acid or oligonucleotide can relate to its hybridization specificity or selectivity.

For analyzing SNPs and other variant nucleic acids, it may be appropriate to use oligonucleotides specific for alternative alleles. Such oligonucleotides which detect single nucleotide variations in target sequences may be referred to by such terms as "allele-specific probes", or "allele-specific primers". The design and use of allele-specific probes for analyzing polymorphisms is described in, e.g., Mutation Detection A Practical Approach, ed. Cotton et al. Oxford University Press, 1998; Saiki et al, Nature, 324: 163-166 (1986); Dattagupta, EP235,726; and Saiki, WO 89/11548. In one embodiment, a probe or primer may be designed to hybridize to a segment of target DNA such that the SNP aligns with either the 5' most end or the 3' most end of the probe or primer.

In some embodiments, the amplification may include a labeled primer, thereby allowing detection of the amplification product of that primer. In particular embodiments, the amplification may include a multiplicity of labeled primers; typically, such primers are distinguishably labeled, allowing the simultaneous detection of multiple amplification products.

In one type of PCR-based assay, an allele-specific primer hybridizes to a region on a target nucleic acid molecule that overlaps a SNP position and only primes amplification of an allelic form to which the primer exhibits perfect complementarity (Gibbs, 1989, Nucleic Acid Res., 17:2427-2448). Typically, the primer's 3'-most nucleotide is aligned with and complementary to the SNP position of the target nucleic acid molecule. This primer is used in conjunction with a second primer that hybridizes at a distal site. Amplification proceeds from the two primers, producing a detectable product that indicates which allelic form is present in the test sample. A control is usually performed with a second pair of primers, one of which shows a single base mismatch at the polymorphic site and the other of which exhibits perfect complementarity to a distal site. The single-base mismatch prevents amplification or substantially reduces amplification efficiency, so that either no detectable product is formed or it is formed in lower amounts or at a slower pace. The method generally works most effectively when the mismatch is at the 3'-most position of the oligonucleotide (i.e., the 3'-most position of the oligonucleotide aligns with the target SNP position) because this position is most destabilizing to elongation from the primer (see, e.g., WO 93/22456).

In a specific embodiment, a primer contains a sequence substantially complementary to a segment of a target SNP-containing nucleic acid molecule except that the primer has a mismatched nucleotide in one of the three nucleotide positions at the 3'-most end of the primer, such that the mismatched nucleotide does not base pair with a particular allele at the SNP site. In one embodiment, the mismatched nucleotide in the primer is the second from the last nucleotide at the 3'-most position of the primer. In another embodiment, the mismatched nucleotide in the primer is the last nucleotide at the 3'-most position of the primer.

In one embodiment, primer or probe is labeled with a fluorogenic reporter dye that emits a detectable signal. While a suitable reporter dye is a fluorescent dye, any reporter dye that can be attached to a detection reagent such as an oligonucleotide probe or primer is suitable for use in the invention. Such dyes include, but are not limited to, Acridine, AMCA, BODIPY, Cascade Blue, Cy2, Cy3, Cy5, Cy7, Dabcyl, Edans, Eosin, Erythrosin, Fluorescein, 6-Fam, Tet, Joe, Hex, Oregon Green, Rhodamine, Rhodol Green, Tamra, Rox, and Texas Red.

It will be appreciated that the invention extends to reagents that do not contain (or that are complementary to) a SNP nucleotide identified herein but that are used to assay one or more SNPs disclosed herein. For example, primers that flank, but do not hybridize directly to a target SNP position provided herein are useful in primer extension reactions in which the primers hybridize to a region adjacent to the target SNP position (i.e., within one or more nucleotides from the target SNP site). During the primer extension reaction, a primer is typically not able to extend past a target SNP site if a particular nucleotide (allele) is present at that target SNP site, and the primer extension product can readily be detected in order to determine which SNP allele is present at the target SNP site. For example, particular ddNTPs are typically used in the primer extension reaction to terminate primer extension once a ddNTP is incorporated into the extension product. Thus, reagents that bind to a nucleic acid molecule in a region adjacent to a SNP site, even though the bound sequences do not necessarily include the SNP site itself, are also encompassed by the invention.

Variant nucleic acids may be amplified prior to detection or may be detected directly during an amplification step (i.e., "real-time" methods). In some embodiments, the target sequence is amplified and the resulting amplicon is detected by electrophoresis. In some embodiments, the specific mutation or variant is detected by sequencing the amplified nucleic acid. In some embodiments, the target sequence is amplified using a labeled primer such that the resulting amplicon is detectably labeled. In some embodiments, the primer is fluorescently labeled.

In one embodiment, detection of a variant nucleic acid, such as a SNP, is performed using the TaqMan® assay, which is also known as the 5' nuclease assay (U.S. Pat. Nos. 5,210,015 and 5,538,848) or Molecular Beacon probe (U.S. Pat. Nos. 5,118,801 and 5,312,728), or other stemless or linear beacon probe (Livak et al, 1995, PCR Method AppL, 4:357-362; Tyagi et al, 1996, Nature Biotechnology, 14:303-308; Nazarenko et al, 1997, Nucl. Acids Res., 25:2516-2521; U.S. Pat. Nos. 5,866,336 and 6,117,635). The TaqMan® assay detects the accumulation of a specific amplified product during PCR. The TaqMan® assay utilizes an oligonucleotide probe labeled with a fluorescent reporter dye and a quencher dye. The reporter dye is excited by irradiation at an appropriate wavelength, it transfers energy to the quencher dye in the same probe via a process called fluorescence resonance energy transfer (FRET). When attached to the probe, the excited reporter dye does not emit a signal. The proximity of the quencher dye to the reporter dye in the intact probe maintains a reduced fluorescence for the reporter. The reporter dye and quencher dye may be at the 5' most and the 3' most ends, respectively or vice versa. Alternatively, the reporter dye may be at the 5 ' or 3' most end while the quencher dye is attached to an internal nucleotide, or vice versa. In yet another embodiment, both the reporter and the quencher may be attached to internal nucleotides at a distance from each other such that fluorescence of the reporter is reduced.

During PCR, the 5' nuclease activity of DNA polymerase cleaves the probe, thereby separating the reporter dye and the quencher dye and resulting in increased fluorescence of the reporter. Accumulation of PCR product is detected directly by monitoring the increase in fluorescence of the reporter dye. The DNA polymerase cleaves the probe between the reporter dye and the quencher dye only if the probe hybridizes to the target SNP- containing template which is amplified during PCR, and the probe is designed to hybridize to the target SNP site only if a particular SNP allele is present.

TaqMan® primer and probe sequences can readily be determined using the variant and associated nucleic acid sequence information provided herein. A number of computer programs, such as Primer Express (Applied Biosystems, Foster City, Calif.), can be used to rapidly obtain optimal primer/probe sets. It will be apparent to one of skill in the art that such primers and probes for detecting the genetic variations of the invention are useful in predictive assays for fracture risk and related pathologies, and can be readily incorporated into a kit format. The invention also includes modifications of the TaqMan® assay well known in the art such as the use of Molecular Beacon probes (U.S. Pat. Nos. 5,118,801 and 5,312,728) and other variant formats (U.S. Pat. Nos. 5,866,336 and 6,117,635).

Other methods of probe hybridization detected in real time can be used for detecting amplification of a target or marker sequence flanking a tandem repeat region. For example, the commercially available MGB Eclipse™ probes (Epoch Biosciences), which do not rely on a probe degradation can be used. MGB Eclipse™ probes work by a hybridization-triggered fluorescence mechanism. MGB Eclipse™ probes have the Eclipse™ Dark Quencher and the MGB positioned at the 5'-end of the probe. The fluorophore is located on the 3'-end of the probe. When the probe is in solution and not hybridized, the three dimensional conformation brings the quencher into close proximity of the fluorophore, and the fluorescence is quenched. However, when the probe anneals to a target or marker sequence, the probe is unfolded, the quencher is moved from the fluorophore, and the resultant fluorescence can be detected.

Oligonucleotide probes can be designed which are between about 10 and about 100 nucleotides in length and hybridize to the amplified region. Oligonucleotides probes are preferably 12 to 70 nucleotides; more preferably 15-60 nucleotides in length; and most preferably 15-25 nucleotides in length. The probe may be labeled. Amplified fragments may be detected using standard gel electrophoresis methods. For example, in preferred embodiments, amplified fractions are separated on an agarose gel and stained with ethidium bromide by methods known in the art to detect amplified fragments.

Another suitable detection methodology involves the design and use of bipartite primer/probe combinations such as Scorpion™ probes. These probes perform sequence- specific priming and PCR product detection is achieved using a single molecule. Scorpion™ probes comprise a 3' primer with a 5' extended probe tail comprising a hairpin structure which possesses a fluorophore/quencher pair. The probe tail is "protected" from replication in the 5' to 3' direction by the inclusion of hexethlyene glycol (HEG) which blocks the polymerase from replicating the probe. The fluorophore is attached to the 5' end and is quenched by a moiety coupled to the 3' end. After extension of the Scorpion™ primer, the specific probe sequence is able to bind to its complement within the extended amplicon thus opening up the hairpin loop. This prevents the fluorescence from being quenched and a signal is observed. A specific target is amplified by the reverse primer and the primer portion of the Scorpion™, resulting in an extension product. A fluorescent signal is generated due to the separation of the fluorophore from the quencher resulting from the binding of the probe element of the Scorpion™ to the extension product. Such probes are described in Whitcombe et al, Nature Biotech 17: 804-807 (1999).

To determine the association of genetic variation (i.e. genotype) with fracture risk, the genotype of subjects with the disease is compared to the genotype of subjects without the disease or simply the genotype of the wild type or native genome.

Once the genotypes of each group are known, the risk of developing fracture risk, or the duration of remission, can be determined statistically. Thus, in one embodiment the method additionally comprises the step of calculating the risk of developing fracture risk.

One such method for calculating the risk is using odds ratios (OR). This widely used statistic compares the retrospective/posterior odds of exposure to a given risk factor in two groups of individuals. The OR can be manually calculated using contingency tables for each genetic variation (i.e. SNP).

In an alternative embodiment, the estimation of risk may be based on multiple SNPs, known as genomic selection and complex trait prediction. Such statistical methods include those described in: Meuwissen et al. (Genetics 157, 1819-1829 (2001)); Shepherd et al. (BMC Bioinformatics 11, 529 (2010)); and Yang et al. (Am. J. Hum. Genet. 88, 76-82 (2011)).

More commonly, a statistical software package may be used, particularly when more than one SNP is being evaluated. Numerous such software packages are available, both commercially and via publicly available websites, such as PLINK (Purcell et al. (2007) Am J Hum Genet 81, 559 - 575).

According to a further aspect of the disclosure there is provided a kit for predicting fracture risk which comprises instructions to use said kit in accordance with the methods defined herein.

The kits may be prepared for practicing the methods described herein. Typically, the kits include at least one component or a packaged combination of components useful for practicing the method of the invention. The kits may include some or all of the components necessary to practice method of the invention. Typically, the kits include at least one probe specific for the one or more regions defined herein in at least one container. These components may include, *inter alia,* nucleic acid probes, nucleic acid primers for amplification of the one or more regions defined herein, buffers, instructions for use, and the like.

The following study illustrates the invention. In particular, a genome-wide association scan was conducted and the molecular heritability for fracture risk in the thoroughbred horse was estimated. The data presented herein confirms the highly heritable and complex nature of fracture risk and identifies several key genomic regions and candidate genes associated with prediction of fracture risk.

### MATERIALS AND METHODS

### Data and samples

Fracture cases (n=269) were horses that sustained catastrophic distal limb fractures on UK racecourses, requiring euthanasia, and were obtained from an archive of samples collected during a previous study between February 1999 and May 2005 (Parkin *et al.* 2004 *supra*). The exact fracture site and type were identified by post-mortem examination. Controls (n=253) were a mixture of uninjured horses originally selected from the same race as the case (n = 66) and uninjured horses sampled as part of a previous study (n=187). All control horses were traced and verified as having no known history of fracture up to the time of the study. Horses sampled were bred for both flat and National Hunt (NH) racing: of the cases, 135 were flat-bred, 110 NH-bred and 24 of unknown status, of the controls 117 were flat-bred, 135 NH-bred and 1 of unknown status (see Table 2).

**Table 2: Distribution of cases and controls by background and sex**

| | **Cases** | **Controls** |
|---|---|---|
| **Flat-bred** | | |
| Male | 104 | 103 |
| Female | 31 | 14 |
| | | |

| **National Hunt (NH)-bred** | | |
|---|---|---|
| Male | 83 | 116 |
| Female | 27 | 19 |
| | | |
| **Unknown status** | | |
| Male | 20 | 1 |
| Female | 4 | - |
| **Total** | 269 | 253 |

### DNA extraction and quantification

Samples consisted of either tissue or bone marrow biopsies (cases) or blood samples collected in EDTA (controls). DNA was extracted using Nucleon BACC DNA extraction kits (http://www.tepnel.com/dna-extraction-kits-blood-and-cell-culture.asp). DNA samples were then quantified in duplicate using Picogreen (http://probes.invitrogen.com/media/pis/mp07581.pdf) and a selection (approximately 10%) were run on agarose gel to check for the presence of undegraded, high-molecular weight DNA. A small dilution of each sample was prepared at 70ng/µl for genotyping.

### SNP genotyping and quality control

SNP genotyping was carried out by Cambridge Genomic Services (http://www.cqs.path.cam.ac.uk/services/snp-genotyping/services.html) using the Illumina EquineSNP50 BeadChip. (www.illumina.com/documents/products/datasheets/datasheet equine snp50.p df). This BeadChip carries 54,602 SNP assays (average spacing 43.2 kb) selected from the database of over one million SNPs (http://www.broadinstitute.org/ftp/distribution/horse snp release/v2/) generated during the sequencing of the horse genome (http://www.broadinstitute.org/mammals/horse).

The genotyping data were analysed with the Illumina GenomeStudio genotyping module (http://www.illumina.com/documents/products/datasheets/datasheet genomest udio software.pdf). A cluster file was generated directly from this dataset (n = 545) and 797 additional thoroughbred samples genotyped at the same time. All genotyping data were clustered *de novo* for the 1,342 samples. The average SNP call frequency was 98.82%, with 150 SNPs not called. Nineteen samples (1.4%) had a call rate less than 95% and these were discarded. The remaining samples were then re-clustered. The average SNP call frequency had increased to 99.17%, with only 143 SNPs (0.26%) not called from the 54,602 on the EquineSNP50 BeadChip.

All SNPs were then subjected to a number of editing steps with GenomeStudio software during which thresholds were applied for a number of metrics following the chip manufacturer's guidelines (http://www.illumina.com/downloads/GTDataAnalysis TechNote.pdf). This resulted in the removal of 190 SNPs with low intensity data (AB R Mean), 1265 SNPs with inadequately defined clusters (cluster separation), 2279 SNPs with call rates less than 98%, 297 SNPs where the heterozygote cluster was not well separated from the homozygote clusters (AB T Mean), 119 SNPs where genotypes differ significantly from Hardy-Weinberg equilibrium and 51 SNPs where X chromosome SNPs were heterozygous in males. A total of 4,201 SNPs were removed, leaving 50,401 for further analysis. The mean call frequency in the remaining SNPs was 99.83%.

Further quality control procedures on the samples, such as estimation of sample gender based on X chromosome genotypes and identification of duplicated samples based on genotype identity, resulted in 39 samples out of 1342 being discarded. From the fracture study samples 269 cases and 253 controls passed the quality control procedures and 7 cases and 16 control samples failed.

### Statistical analysis

Association and logistic regression analyses were performed using PLINK (Purcell et al. 2007 Am. J. Hum. Genet. 81, 559-575). Markers with a minor allele frequency (MAF) less than 2% were excluded from the analysis (n=11,124), as were markers that failed the Hardy-Weinberg equilibrium test (p < 0.001) (n=96), and markers with more than 10% of genotypes missing (n=4,223): 43,417 SNPs remained in the analysis. Possible population stratification was assessed by calculating identity-by-state (IBS) sharing among all pairs of individuals (Figure 1). The Cochran-Mantel-Haenszel (CMH) association test was used in order to correct for population stratification (ppc 0.001). A single SNP association was carried out, with *p*-values adjusted for multiple testing using 1,000 permutations.

66 additional SNPs were added to ECA 18 between 61.85 Mb and 72.87 Mb (described in the methodology hereinbefore and Table 3).

**Table 3: Known Genes in the equine fracture risk genome regions and corresponding homologous human genes**

| **ECA 18: 61984390 - 66495180 syntenic to HSA 2: 185463093 - 190927455** | | | |
|---|---|---|---|
| *Equus caballus Genes* | | *Homo sapiens Homologues* | |
| ID | Location | ID | Location |
| ZNF804A (ENSECAG00000011071) | 18:61984390-62047629 | ZNF804A (ENSG00000170396) | 2:185463093-185804219 |
| LOC100068553 (ENSECAG00000013931) | 18:62733459-62767978 | No homologues | |
| LOC100068571 (ENSECAG00000014156) | 18:62770871-62775811 | FSIP2 (ENSG00000188738) | 2:186603355-186698017 |
| LOC100068571 (ENSECAG00000015082) | 18:62781591-62806979 | No homologues | |
| LOC100068634 (ENSECAG00000017149) | 18:63322282-63344464 | ZC3H15 (ENSG00000065548) | 2:187350883-187374090 |
| B0I1H0_HORSE (ENSECAG00000022653) | 18:63417718-63498704 | ITGAV (ENSG00000138448) | 2:187454790-187545628 |
| LOC100054234 (ENSECAG00000021377) | 18:63514629-63575391 | FAM171B (ENSG00000144369) | 2:187558698-187630685 |
| LOC100068760 (ENSECAG00000007054) | 18:63609890-63629100 | ZSWIM2 (ENSG00000163012) | 2:187692562-187713935 |
| ENSECAG00000003166 (ENSECAG00000003166) | 18:64034370-64035194 | No homologues | |
| LOC100054281 (ENSECAG00000012990) | 18:64065062-64102603 | CALCRL (ENSG00000064989) | 2:188207856-188313187 |
| TFPI (ENSECAG00000021849) | 18:64189190-64264609 | TFPI (ENSG00000003436) | 2:188328957-188430487 |
| ENSECAG00000003186 (ENSECAG00000003186) | 18:64696394-64696798 | No homologues | |
| LOC100054329 (ENSECAG00000023352) | 18:65110394-65173364 | GULP1 (ENSG00000144366) | 2:189156396-189460653 |
| ENSECAG00000024768 (ENSECAG00000024768) | 18:65138951-65139448 | No homologues | |
| O97950_HORSE (ENSECAG00000024769) | 18:65487247-65526274 | COL3A1 (ENSG00000168542) | 2:189839046-189877472 |
| LOC100054421 (ENSECAG00000008499) | 18:65549357-65689370 | COL5A2 (ENSG00000204262) | 2:189896622-190044605 |
| ENSECAG00000003220 (ENSECAG00000003220) | 18:65771552-65772073 | No homologues | |
| LOC100069122 (ENSECAG00000010710) | 18:65958277-65984553 | WDR75 (ENSG00000115368) | 2:190306159-190340291 |
| SLC40A1 (ENSECAG00000021609) | 18:66076610-66096537 | SLC40A1 (ENSG00000138449) | 2:190425305-190448484 |
| ASNSD1 (ENSECAG00000024547) | 18:66182057-66189594 | ASNSD1 (ENSG00000138381) | 2:190526146-190535557 |
| ANKAR (ENSECAG00000003846) | 18:66194788-66258045 | ANKAR (ENSG00000151687) | 2:190539016-190625919 |
| OSGEPL1 (ENSECAG00000011795) | 18:66261695-66270443 | OSGEPL1 (ENSG00000128694) | 2:190611386-190627953 |
| ORMDL1 (ENSECAG00000013119) | 18:66280052-66291854 | ORMDL1 (ENSG00000128699) | 2:190635049-190649097 |
| PMS1 (ENSECAG00000013591) | 18:66297947-66381649 | PMS1 (ENSG00000064933) | 2:190649107-190742355 |
| MSTN (ENSECAG00000021373) | 18:66490208-66495180 | MSTN (ENSG00000138379) | 2:190920423-190927455 |

| **ECA 22: 38581665 - 39875629 syntenic to HSA 20: 49126891 - 49201299** | | | |
|---|---|---|---|
| *Equus caballus Genes* | | *Homo sapiens Homologues* | |
| ID | Location | ID | Location |
| LOC100052066 (ENSECAG00000019772) | 22:38581665-38602458 | PTPN1 (ENSG00000196396) | 20:49126891-49201299 |
| LOC100049830 (ENSECAG00000004962) | 22:38606641-38635889 | FAM65C (ENSG00000042062) | 20:49202645-49308065 |
| LOC100052187 (ENSECAG00000009294) | 22:38713058-38722759 | PARD6B (ENSG00000124171) | 20:49348081-49373332 |
| LOC100049902 (ENSECAG00000009307) | 22:38814231-38845063 | ADNP (ENSG00000101126) | 20:49505585-49547750 |
| LOC100052367 (ENSECAG00000011277) | 22:38850823-38872525 | DPM1 (ENSG00000000419) | 20:49551404-49575092 |
| LOC100052426 (ENSECAG00000004476) | 22:38872823-38874288 | MOCS3 (ENSG00000124217) | 20:49575363-49577820 |
| LOC100052484 (ENSECAG00000017924) | 22:38921062-38926932 | KCNG1 (ENSG00000026559) | 20:49620193-49639666 |
| LOC100052657 (ENSECAG00000018997) | 22:39230517-39368456 | NFATC2 (ENSG00000101096) | 20:50003494-50179339 |
| LOC100052707 (ENSECAG00000020949) | 22:39416403-39511356 | ATP9A (ENSG00000054793) | 20:50213053-50385173 |
| SALL4 (ENSECAG00000018533) | 22:39550017-39566556 | SALL4 (ENSG00000101115) | 20:50400581-50419014 |
| ZFP64 (ENSECAG00000021570) | 22:39796475-39875629 | ZFP64 (ENSG00000020256) | 20:50668202-50820847 |
| | | | |

| **ECA 1: 13592262 - 15006221 syntenic to HSA 10: 120433679 - 118969810** | | | |
|---|---|---|---|
| *Equus caballus Genes* | | *Homo sapiens Homologues* | |
| ID | Location | ID | Location |
| LOC100058684 (ENSECAG00000013139) | 1:13592262-13654539 | C10orf46 (ENSG00000151893) | 10:120433679-120514761 |
| LOC100066425 (ENSECAG00000003701) | 1:13735786-13736973 | PRLHR (ENSG00000119973) | 10:120352916-120355160 |
| LOC100058723 (ENSECAG00000019024) | 1:13975012-13999484 | C10orf84 (ENSG00000165669) | 10:120065401-120101840 |
| LOC100058765 (ENSECAG00000019791) | 1:14244165-14281080 | RAB11FIP2 (ENSG00000107560) | 10:119764427-119806114 |
| LOC100067044 (ENSECAG00000021102) | 1:14689686-14694378 | EMX2 (ENSG00000170370) | 10:119301956-119309057 |
| PDZD8 (ENSECAG00000023468) | 1:14843299-14926474 | PDZD8 (ENSG00000165650) | 10:119040000-119134978 |
| LOC100058805 (ENSECAG00000024054) | 1:14931854-14966735 | SLC18A2 (ENSG00000165646) | 10:119000604-119038941 |
| LOC100058839 (ENSECAG00000022730) | 1:14993935-15006221 | KCNK18 (ENSG00000186795) | 10:118957000-118969810 |
| | | | |

| **ECA 9: 51425562 - 54321719 syntenic to HSA 8: 107282473 - 107764922** | | | |
|---|---|---|---|
| *Equus caballus Genes* | | *Homo sapiens Homologues* | |
| ID | Location | ID | Location |
| OXR1 (ENSECAG00000022434) | 9:51425562-51505321 | AC090579.1 (ENSG00000164830) | 8:107282473-107764922 |
| ABRA (ENSECAG00000015998) | 9:51517001-51526650 | ABRA (ENSG00000174429) | 8:107771711-107782473 |
| LOC100056482 (ENSECAG00000016669) | 9:51942203-52181014 | ANGPT1 (ENSG00000154188) | 8:108261721-108510283 |
| A8C9U1_HORSE (ENSECAG00000024853) | 9:52480497-52626021 | RSPO2 (ENSG00000147655) | 8:108911544-109095913 |
| LOC100063841 (ENSECAG00000000236) | 9:52702461-52751104 | EIF3E (ENSG00000104408) | 8:109213445-109447562 |
| LOC100063908 (ENSECAG00000010199) | 9:52918484-52978069 | TTC35 (ENSG00000104412) | 8:109455830-109499145 |
| LOC100056570 (ENSECAG00000005498) | 9:53279396-53280313 | TMEM74 (ENSG00000164841) | 8:109619079-109799844 |
| LOC100056618 (ENSECAG00000023156) | 9:53530152-53564683 | TRHR (ENSG00000174417) | 8:110098850-110131813 |
| NUDCD1 (ENSECAG00000023445) | 9:53724876-53755532 | NUDCD1 (ENSG00000120526) | 8:110253148-110346614 |
| LOC100056701 (ENSECAG00000023565) | 9:53755783-53765059 | ENY2 (ENSG00000120533) | 8:110346553-110358182 |
| LOC100064208 (ENSECAG00000000972) | 9:53782588-53933100 | PKHD1L1 (ENSG00000205038) | 8:110374706-110542559 |
| LOC100064267 (ENSECAG00000009719) | 9:53953266-53973218 | EBAG9 (ENSG00000147654) | 8:110551940-110578225 |
| ENSECAG00000005533 (ENSECAG00000005533) | 9:53968315-53969139 | No homologues | |
| LOC100064295 (ENSECAG00000012812) | 9:53983702-54091295 | SYBU (ENSG00000147642) | 8:110586207-110704020 |
| LOC100064355 (ENSECAG00000019378) | 9:54315947-54321719 | KCNV1 (ENSG00000164794) | 8:110975874-110988076 |
| | | | |

| **ECA 21: 55530918 - 56975262 syntenic to HSA 5: 2745959 - 1039058** | | | |
|---|---|---|---|
| *Equus caballus Genes* | | *Homo sapiens Homologues* | |
| ID | Location | ID | Location |
| LOC100071850 (ENSECAG00000015758) | 21:55530918-55533148 | IRX2 (ENSG00000170561) | 5:2745959-2752969 |
| LOC100147154 (ENSECAG00000017403) | 21:56312150-56316470 | IRX4 (ENSG00000113430) | 5:1877541-1887350 |
| LOC100071879 (ENSECAG00000021690) | 21:56388290-56396533 | NDUFS6 (ENSG00000145494) | 5:1801514-1816719 |
| LOC100071885 (ENSECAG00000001222) | 21:56398975-56399256 | MRPL36 (ENSG00000171421) | 5:1798500-1801480 |
| LOC100071898 (ENSECAG00000021716) | 21:56626299-56657804 | LPCAT1 (ENSG00000153395) | 5:1456595-1524092 |
| SLC6A3 (ENSECAG00000024615) | 21:56678340-56714116 | SLC6A3 (ENSG00000142319) | 5:1392909-1445545 |
| CLPTM1L (ENSECAG00000013231) | 21:56747297-56765133 | CLPTM1L (ENSG00000049656) | 5:1317859-1345214 |
| TERT (ENSECAG00000001347) | 21:56785618-56802579 | TERT (ENSG00000164362) | 5:1253262-1295184 |
| LOC100058040 (ENSECAG00000009911) | 21:56924692-56958209 | SLC12A7 (ENSG00000113504) | 5:1050499-1112172 |
| LOC100057994 (ENSECAG00000016907) | 21:56969766-56975262 | NKD2 (ENSG00000145506) | 5:1008944-1039058 |

Haplotype blocks in the regions of interest on ECA 18 and 22 were identified based on the value of r² using HAPLOVIEW (Barrett et al. (2005) Bioinformatics 21, 263-265). Blocks containing significant SNPs were further analysed using haplotype logistic regression, with sex and flat/National Hunt-bred fitted as co-variates. Corrected *p*-values for the logistic regressions were obtained with 10,000 permutations. Haplotype frequencies within cases and controls were also determined.

Molecular estimates of heritability and genetic variance explained overall and by individual chromosomes were obtained using GCTA (Yang et al. (2011) Am. J. Hum. Genet. 88, 76-82). The genetic relationship matrix was derived from the 43,417 genotyped SNPs and sex was fitted as a fixed effect in the REML analysis.

### RESULTS

High density SNP genotyping was used to carry out a genome-wide association scan and to estimate the molecular heritability for fracture risk in the thoroughbred horse. A total of 54,666 SNPs were genotyped (54,588 SNPs with the Illumina Equine SNP50 BeadChip and an additional 78 SNPs on ECA 18 (66 of which passed QC procedures) with a custom Illumina GoldenGate assay) on 269 cases and 253 controls. Cases were horses that had suffered a catastrophic distal limb fracture while racing. Controls were animals that were over 4 years of age and had no history of fracture at the time the study was carried out. After filtering of SNPs for genotyping quality, minor allele frequency > 0.02 and Hardy-Weinberg equilibrium 43,417 SNPs remained in the analysis. Significant stratification was present in the sample as horses bred for both flat and National Hunt racing were included (IBS test *p* < 1 x 10⁻⁵). Association testing was carried out using the Cochran-Mantel-Haenszel (CMH) test, in order to account for population stratification.

Four SNPs, three on ECA 18 and one on ECA 1, reached genome-wide significance after correction for multiple testing (*p*_{genome} < 0.05) (see Table 4).

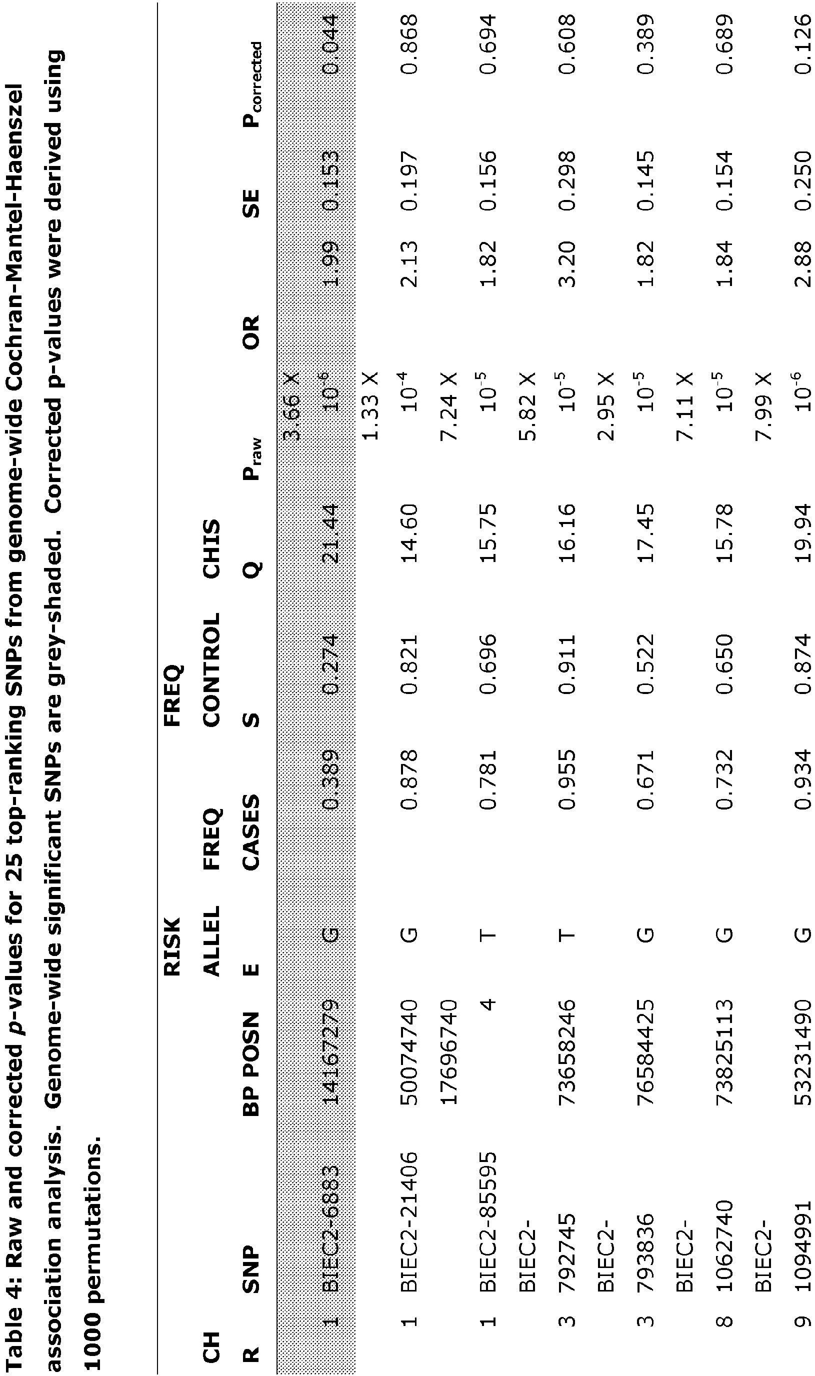

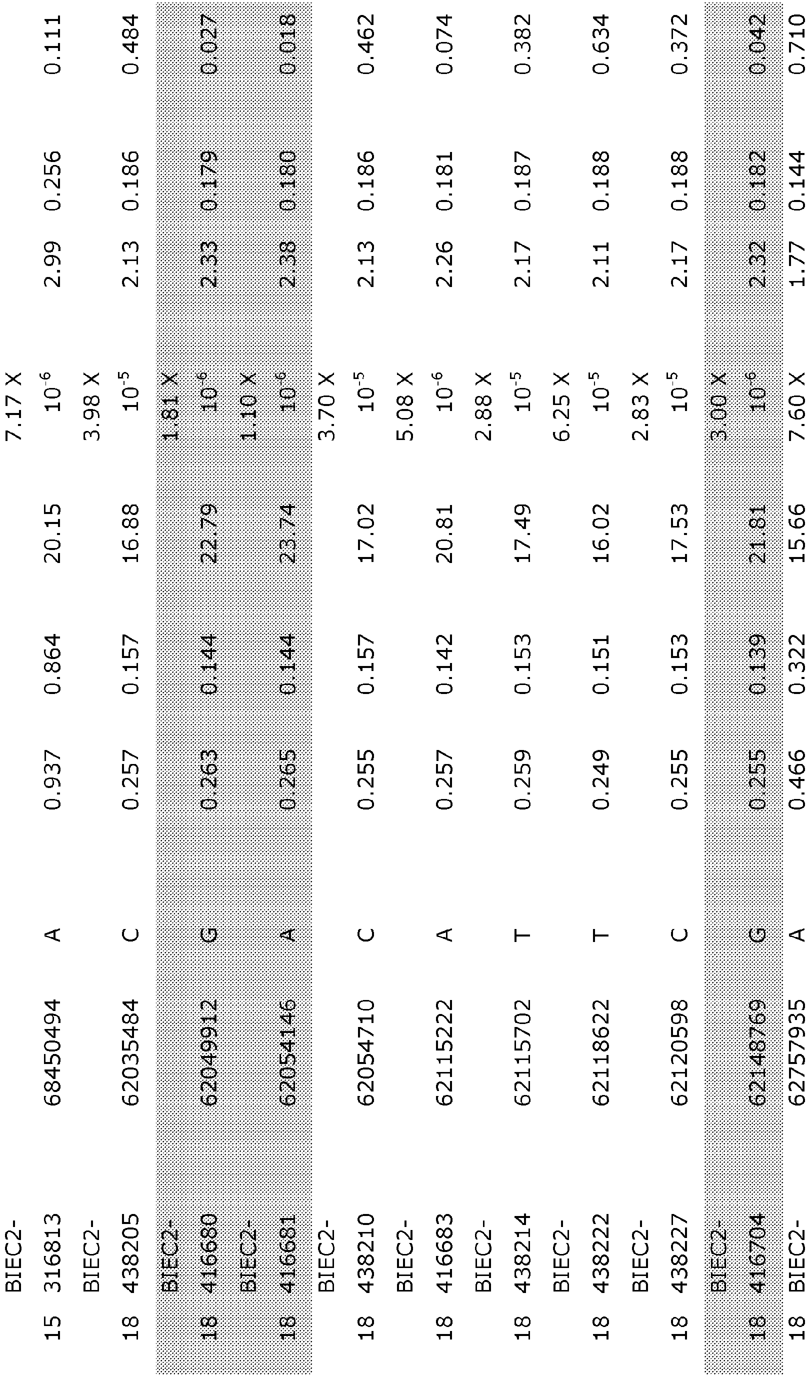

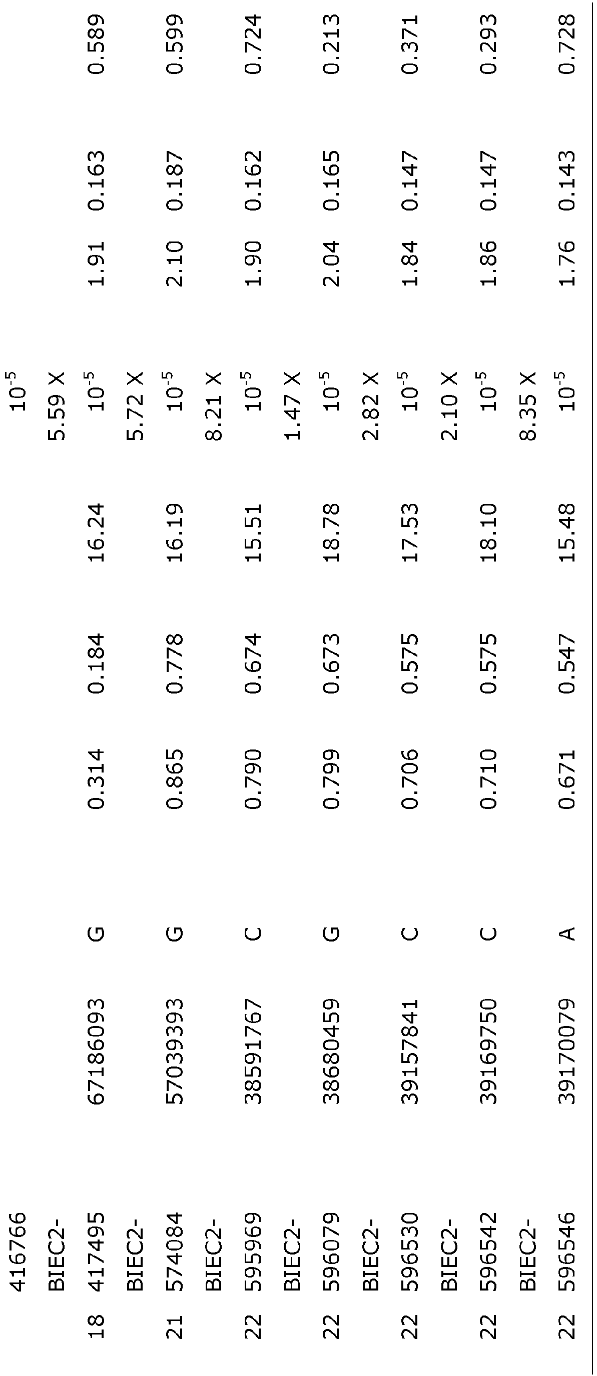

ECA 18 (*p*_{genome} = 0.018) showed evidence for more than one SNP associated with distal limb fracture. A number of supporting SNPs are seen, with the peak localizing to around 62 Mb. There is also evidence of suggestive signals seen on ECA 3, 8, 9, 15, 21 and 22 although they do not reach genome-wide significance level. Figure 2 (A) shows a Manhattan plot of the raw *p*-values from the genome-wide association (Cochran-Mantel-Haenszel test) scan for catastrophic distal limb fracture (B) empirical p-values calculated after 1000 permuations (C) empirical p-values for ECA 18 plotted against SNP position on the chromosome (Mb).

Examination of the linkage disequilibrium (LD) among markers showed that the most significant SNPs on ECA 18 fall into an LD block containing 10 SNPs in total and spanning 140 kb (haplotype block 1 in Figure 3). All SNPs within the block are in high LD with each other with pairwise r² of at least 0.8. The haplotype GGAGGCTAAA is at higher frequency in the controls and has a protective effect, with logistic regression (Table 5) showing that controls are 1.95 times at less risk of fracture than cases (*p* = 1 x 10⁻⁴).

TGGAATTAAG, a risk haplotype, is at low frequency in the cases and, in this data set, absent from the controls. Cases with this haplotype are at 3.39 times higher risk of fracture than controls (*p* = 0.042).

There is low LD (r² < 0.1) between adjacent haplotype blocks in the ECA 18 region. Haplotype block 1 contains 39.5 kb of the gene ZNF804A and the two most significant SNPs are located 2.2 kb from the end of the gene. ZNF804A has been reported to have a variant associated with schizophrenia in humans (O'Donovan et al. (2008) Nat. Genet. 40, 1053-1055; Esslinger et al. (2009) Science 324, 605) and regulates expression of genes such as the catechol O-methyl transferase gene (*COMT*) (Girgenti et al. (2012) PLoS ONE 7, e32404) which has been associated with increased fracture risk in males (Erikkson et al. (2008) Bone 42, 107-112) .

**Table 5: Logistic regression results for ECA 18 haplotype block 1. Background of horse (flat or National Hunt-bred) and sex were fitted as covariates in the haplotype logistic regression option in PLINK, p-values were derived using 10,000 permutations.**

| **HAPLOTYPE** | **Odds Ratio** | **t-statistic** | **P** | **FREQ CASES** | **FREQ CONTROLS** |
|---|---|---|---|---|---|
| GGAGGCTAAA | 0.511 | 15 | 1 x 10⁻⁴ | 0.738 | 0.856 |
| TAGAACCGGG | 1.42 | 3.66 | 0.161 | 0.196 | 0.130 |
| TGGAATTAAG | 3.39 | 5.41 | 0.042 | 0.029 | 0 |
| GAGAACCGGG | 1.8 x 10⁻¹¹ | 2.51 | 0.286 | 0.024 | 0 |

Other candidate genes in ECA 18 haplotype block 3 are ITGAV (18: 63,417,718 - 63,498,794 a receptor binding to a variety of extracellular matrix proteins including osteopontin and bone sialoprotein), CALCRL (18: 64,065062 - 64, 102,603 calcitonin-like receptor), COL3A1 (18: 65,487,247 - 65,526,274), COL3A2, COL5A2 (18: 65,549,357 - 65,689,370 collagens). LD between ECA 18 haplotype blocks 1 and 3 is generally low, apart from SNPs BIEC2-417210 and BIEC2-417274 which are in moderate LD (r² = 0.35 - 0.44) with SNPs in block 1. The LD may have arisen due to a combination of selected alleles at different genes in this region. For example, there is evidence that racing performance and optimal racing distance in the thoroughbred is influenced by the nearby myostatin (MSTN) locus (Hill et al. (2010) BMC Genomics 11, 552; Hill et al. (2010) PLoS ONE 5, e8645; Tozaki et al. (2010) Animal Genetics 41, Suppl 2, 28-35) and the extent of LD seen in this region may be the result of a selective sweep. However, the SNPs most significantly associated with fracture risk are located in ECA 18 haplotype block 1, strongly suggesting ZNF804A as a candidate gene for fracture risk.

Genetic variance explained by SNPs for fracture risk was estimated to be 0.479 (s.e. 0.124). A log-likelihood of 110.6 for the full model compared with a log-likelihood of 103.4 for the null model (genetic variance σ_{g}² = 0) and likelihood ratio test (LRT) of 14.32 (*p* = 0.00015) confirms the variance is significantly different from zero. Genetic variance estimates for each individual chromosome showed significant variance on chromosomes 9, 18, 22 and 31 (see Figure 4 and Table 6).

**Table 6: Genetic variance and heritability estimates for fracture risk by chromosome. Model fitted includes as co-variates sex and first 20 eigenvectors (to account for population stratification). Estimate of Vp is 0.236 (s.e. 0.015).**

| ***Chromosome*** | ***Va*** | ***S.E.*** | ***h²*** | ***S.E.*** | ***LogL*** | ***LRT*** |
|---|---|---|---|---|---|---|
| 1 | 0.000 | 0.009 | 0.000 | 0.037 | 103.39 | |
| 2 | 0.015 | 0.011 | 0.061 | 0.046 | 104.45 | |
| 3 | 0.005 | 0.009 | 0.023 | 0.039 | 103.56 | |
| 4 | 0.003 | 0.009 | 0.012 | 0.037 | 103.43 | |
| 5 | 0.004 | 0.008 | 0.015 | 0.034 | 103.51 | |
| 6 | 0.000 | 0.006 | 0.000 | 0.027 | 103.39 | |
| 7 | 0.000 | 0.007 | 0.000 | 0.029 | 103.39 | |
| 8 | 0.016 | 0.012 | 0.065 | 0.049 | 104.23 | |
| 9 | 0.025 | 0.013 | 0.101 | 0.050 | 106.91 | 7.04 (*p* = 0.008) |
| 10 | 0.010 | 0.009 | 0.042 | 0.039 | 104.14 | |
| 11 | 0.000 | 0.006 | 0.000 | 0.027 | 103.39 | |
| 12 | 0.000 | 0.006 | 0.000 | 0.026 | 103.39 | |
| 13 | 0.000 | 0.006 | 0.000 | 0.025 | 103.39 | |
| 14 | 0.008 | 0.008 | 0.033 | 0.015 | 104.39 | |
| 15 | 0.012 | 0.010 | 0.051 | 0.042 | 104.35 | |
| 16 | 0.005 | 0.008 | 0.023 | 0.036 | 103.61 | |
| 17 | 0.012 | 0.009 | 0.046 | 0.039 | 104.33 | |
| 18 | 0.021 | 0.011 | 0.087 | 0.044 | 107.27 | 7.76 (*p* = 0.005) |
| 19 | 0.000 | 0.006 | 0.000 | 0.025 | 103.39 | |
| 20 | 0.004 | 0.007 | 0.016 | 0.028 | 103.59 | |
| 21 | 0.012 | 0.009 | 0.052 | 0.036 | 104.97 | 3.16 (*p* = 0.075) |
| 22 | 0.012 | 0.008 | 0.051 | 0.034 | 105.35 | 3.92 (*p* = 0.048) |
| 23 | 0.006 | 0.008 | 0.027 | 0.032 | 103.85 | |
| 24 | 0.000 | 0.006 | 0.000 | 0.028 | 103.36 | |
| 25 | 0.013 | 0.009 | 0.054 | 0.037 | 105.19 | 3.60 (*p* = 0.058) |
| 26 | 0.005 | 0.006 | 0.020 | 0.026 | 103.87 | |
| 27 | 0.000 | 0.006 | 0.000 | 0.028 | 103.39 | |
| 28 | 0.004 | 0.007 | 0.018 | 0.028 | 103.65 | |
| 29 | 0.000 | 0.005 | 0.000 | 0.022 | 103.39 | |
| 30 | 0.000 | 0.005 | 0.000 | 0.019 | 103.39 | |
| 31 | 0.017 | 0.009 | 0.072 | 0.038 | 106.93 | 7.08 (*p* = 0.008) |
| X | 0.000 | 0.007 | 0.000 | 0.031 | 103.39 | |

Chromosomes 9 and 18 accounted for the largest genetic variance, around 19%, followed by chromosomes 22 and 31. Together these chromosomes account for 61.8% of the total estimated genetic variance.

The chromosomal heritability estimates correspond with some, but not all, of the regions identified in the genome-wide association scan. This confirms the difficulty of identifying individual genetic variants underlying fracture risk, and the need for very large sample sizes if individual variants are to be successfully mapped. However, of more immediate interest is the possibility of calculating the genetic risk of individuals and, in thoroughbred breeding, incorporating the information into breeding decisions in order to breed a sounder, more robust racehorse. It is clear that for fracture risk, accurate estimation of genetic risk for an individual will need to be based on genome-wide markers and, for example, REML-based methods to obtain BLUP estimates of risk (Yang *et al.* (2011) *supra).*

## Claims

1. A method of predicting fracture risk in a horse which comprises detecting in a biological sample from the subject one or more genetic variations within one or more of the following regions corresponding to the equine genome:
between 13.1 Mb and 15.1 Mb on chromosome 1; and/or
between 51.4 Mb and 54.4 Mb on chromosome 9; and/or
between 61.0 Mb and 67.2 Mb on chromosome 18; and/or
between 55.5 Mb and 57.8 Mb on chromosome 21; and/or
between 38.5 Mb and 39.9 Mb on chromosome 22;
wherein the presence of such genetic variations is indicative of a positive prediction for the likelihood of fracture risk.

2. The method as defined in claim 1, wherein the genetic variations include: mutations (e.g. point mutations), substitutions, deletions, single nucleotide polymorphisms (SNPs), haplotypes, chromosome abnormalities, Copy Number Variation (CNV), epigenetics and DNA inversions.

3. The method as defined in claim 1 or claim 2, wherein the genetic variation is:
a SNP present on chromosome 1 which is BIEC2-6883; and/or
a SNP present on chromosome 9 which is BIEC2-1094991; and/or
a SNP present on chromosome 18 selected from BIEC2-438205, BIEC2-416680, BIEC2-416681, BIEC2-438210, BIEC2-416683, BIEC2-438214, BIEC2-438222, BIEC2-438227, BIEC2-416704 or BIEC2-416766.

4. The method as defined in any one of claims 1 to 3, wherein the genetic variation is a SNP present on chromosome 18 selected from BIEC2-416680, BIEC2-416681 or BIEC2-416704.

5. The method as defined in any one of claims 1 to 4, wherein the genetic variation is a SNP present on chromosome 18 which is BIEC2-417495.

6. The method as defined in any one of claims 1 to 5, wherein the subject is a horse and the genetic variations are within one or more of the following genes: ZNF804A, FSIP2, ITGAV, CALCRL, COL3A1, COL3A2, COL5A2 or MSTN located between 61.7 Mb and 66.5 Mb on chromosome 18 of the equine genome.

7. The method as defined in claim 6, wherein the genetic variations are within ZNF804A located between 61.7 Mb and 62.1 Mb on chromosome 18 of the equine genome.

8. The method as defined in claim 6 or claim 7, wherein the genetic variations within ZNF804A are selected from:
one or more of the SNPs listed in Table 1; and/or
the SNP within Exon 4 of ZNF804A at base pair position 62045643 of chromosome 18; and/or
the 3'-UTR of ZNF804A selected from those listed in Table 1 at base pair positions 62047178, 62047184, 62047262 and 62047293 of chromosome 18.

9. The method as defined in claim 6, wherein the genetic variations are within MSTN located between 61.7 Mb and 66.5 Mb on chromosome 18 of the equine genome, such as at base pair position 66493737 of chromosome 18.

10. The method as defined in any one of claims 1 to 9, wherein the genetic variation is:
a SNP present on chromosome 21 which is BIEC2-574084; and/or
a SNP present on chromosome 22 selected from BIEC2-595969, BIEC2-596079, BIEC2-596530, BIEC2-596542 or BIEC2-596546.

11. The method as defined in any one of claims 1 to 10, wherein the genetic variations are within ZFP64 located between 39.7 Mb and 39.9 Mb on chromosome 22 of the equine genome.

12. The method as defined in claim 1 wherein the biological sample is selected from: whole blood, blood serum, plasma, urine, saliva, or other bodily fluid (stool, tear fluid, synovial fluid, sputum), hair, cerebrospinal fluid (CSF), or an extract or purification therefrom, or dilution thereof, tissue homogenates, tissue sections and biopsy specimens.

13. method as defined in any one of claims 1 to 12 wherein the detection step comprises one or more of the following techniques: polymerase chain reaction (PCR), reverse transcriptase polymerase chain reaction (RT-PCR), nested PCR, ligase chain reaction, branched DNA signal amplification, amplifiable RNA reporters, Q-beta replication, transcription-based amplification, boomerang DNA amplification, strand displacement activation, cycling probe technology or isothermal nucleic acid sequence based amplification (NASBA), Invader Technology, or other sequence replication assays or signal amplification assays.

14. The method as defined in any one of claims 1 to 13 which additionally comprises the step of calculating susceptibility to fracture risk.

## Patentansprüche

1. Verfahren zur Vorhersage des Risikos von Knochenbrüchen in Pferden, wobei das Verfahren das Detektieren einer oder mehrerer genetischer Variationen in einer oder mehreren der folgenden dem Pferde-Genom entsprechenden Regionen in einer biologischen Probe des Probanden umfasst:
zwischen 13,1 Mb und 15,1 Mb auf Chromosom 1; und/oder
zwischen 51,4 MB und 54,4 Mb auf Chromosom 9; und/oder
zwischen 61,0 Mb und 67,2 Mb auf Chromosom 18; und/oder
zwischen 55,5 Mb und 57,8 Mb auf Chromosom 21; und/oder
zwischen 38,5 Mb und 39,9 Mb auf Chromosom 22;
wobei das Vorhandensein derartiger genetischer Variationen auf eine positive Vorhersage der Wahrscheinlichkeit eines Knochenbruchrisikos hinweist.

2. Verfahren nach Anspruch 1, wobei die genetischen Variationen Folgendes beinhalten: Mutationen (z. B. Punktmutationen), Substitutionen, Deletionen, Einzelnukleotid-Polymorphismen (single nucleotide polymorphisms - SNPs), Haplotypen, Chromosomaberrationen, Kopienzahlvariation (Copy Number Variation - CNV), Epigenetik und DNA-Inversionen.

3. Verfahren nach Anspruch 1 oder Anspruch 2, wobei es sich bei der genetischen Variation um Folgendes handelt:
einen SNP, der auf Chromosom 1 vorliegt und bei dem es sich um BIEC2-6883 handelt; und/oder
einen SNP, der auf Chromosom 9 vorliegt und bei dem es sich um BIEC2-1094991 handelt; und/oder
einen SNP, der auf Chromosom 18 vorliegt und aus BIEC2-438205, BIEC2-416680, BIEC2-416681, BIEC2-438210, BIEC2-416683, BIEC2-438214, BIEC2-438222, BIEC2-438227, BIEC2-416704 oder BIEC2-416766 ausgewählt ist.

4. Verfahren nach einem der Ansprüche 1 bis 3, wobei es sich bei der genetischen Variation um einen SNP handelt, der auf Chromosom 18 vorliegt und aus BIEC2-416680, BIEC2-416681 oder BIEC2-416704 ausgewählt ist.

5. Verfahren nach einem der Ansprüche 1 bis 4, wobei es sich bei der genetischen Variation um einen SNP handelt, der auf Chromosom 18 vorliegt und bei dem es sich um BIEC2-417495 handelt.

6. Verfahren nach einem der Ansprüche 1 bis 5, wobei der Proband ein Pferd ist und die genetischen Variationen in einem oder mehreren der folgenden Gene vorliegen:
ZNF804A, FSIP2, ITGAV, CALCRL, COL3A1, COL3A2, COL5A2 oder MSTN, positioniert zwischen 61,7 Mb und 66,5 Mb auf Chromosom 18 des Pferde-Genoms.

7. Verfahren nach Anspruch 6, wobei die genetischen Variationen in ZNF804A, positioniert zwischen 61,7 Mb und 62,1 Mb auf Chromosom 18 des Pferde-Genoms, vorliegen.

8. Verfahren nach Anspruch 6 oder Anspruch 7, wobei die genetischen Variationen in ZNF804A aus Folgendem ausgewählt sind:
einem oder mehreren der in Tabelle 1 aufgelisteten SNPs; und/oder
dem SNP in Exon 4 von ZNF804A bei Basenpaarposition 62045643 von Chromosom 18; und/oder
dem 3'-UTR von ZNF804A, ausgewählt aus den in Tabelle 1 aufgelisteten, bei den Basenpaarpositionen 62047178, 62047184, 62047262 und 62047293 von Chromosom 18.

9. Verfahren nach Anspruch 6, wobei die genetischen Variationen in MSTN, positioniert zwischen 61,7 Mb und 66,5 Mb auf Chromosom 18 des Pferde-Genoms, wie etwa bei Basenpaarposition 66493737 von Chromosom 18, vorliegen.

10. Verfahren nach einem der Ansprüche 1 bis 9, wobei es sich bei der genetischen Variation um Folgendes handelt:
einen SNP, der auf Chromosom 21 vorliegt und bei dem es sich um BIEC2-574084 handelt; und/oder
einen SNP, der auf Chromosom 22 vorliegt und aus BIEC2-595969, BIEC2-596079, BIEC2-596530, BIEC2-596542 oder BIEC2-596546 ausgewählt ist.

11. Verfahren nach einem der Ansprüche 1 bis 10, wobei die genetischen Variationen in ZFP64, positioniert zwischen 39,7 Mb und 39,9 Mb auf Chromosom 22 des Pferde-Genoms, vorliegen.

12. Verfahren nach Anspruch 1, wobei die biologische Probe aus Folgendem ausgewählt ist: Vollblut, Blutserum, Plasma, Urin, Speichel oder einer anderen Körperflüssigkeit (Stuhl, Tränenflüssigkeit, Synovialflüssigkeit, Sputum), Haar, Zerebrospinalflüssigkeit (cerebrospinal fluid - CSF) oder einem Extrakt oder gereinigten Produkt davon oder einer Verdünnung davon, Gewebehomogenaten, Gewebeschnitten und Biopsieproben.

13. Verfahren nach einem der Ansprüche 1 bis 12, wobei der Detektionsschritt eine oder mehrere der folgenden Techniken umfasst: Polymerase-Kettenreaktion (polymerase chain reaction - PCR), Reverse-Transkriptase-Polymerase-Kettenreaktion (RT-PCR), verschachtelte PCR, Ligase-Kettenreaktion, Signalamplifikation verzweigter DNA, amplifizierbaren RNA-Reportern, Q-beta-Replikation, Amplifikation auf Transkriptionsbasis, Bumerang-DNA-Amplifikation, Strangverdrängungsaktivierung, Zyklussonden-Technologie oder isothermer Amplifikation auf Nukleinsäuresequenz-Basis (nucleic acid sequence based amplification - NASBA), Invader-Technologie oder anderen Sequenzreplikationstestverfahren oder Signalamplifikationstestverfahren.

14. Verfahren nach einem der Ansprüche 1 bis 13, das zusätzlich den Schritt des Berechnens der Anfälligkeit für ein Knochenbruchrisiko umfasst.

## Revendications

1. Méthode destinée à prédire le risque de fracture chez un cheval, qui comprend la détection, dans un échantillon biologique du sujet, d'une ou plusieurs variations génétiques dans l'une ou plusieurs des régions suivantes correspondant au génome équin :
entre 13,1 Mb et 15,1 Mb sur le chromosome 1 ; et/ou
entre 51,4 Mb et 54,4 Mb sur le chromosome 9 ; et/ou
entre 61,0 Mb et 67,2 Mb sur le chromosome 18 ; et/ou
entre 55,5 Mb et 57,8 Mb sur le chromosome 21 ; et/ou
entre 38,5 Mb et 39,9 Mb sur le chromosome 22 ;
dans laquelle la présence de ces variations génétiques est indicative d'une prévision positive de la probabilité du risque de fracture.

2. Méthode selon la revendication 1, dans laquelle les variations génétiques incluent : des mutations (par exemple, des mutations ponctuelles), des substitutions, des délétions, des polymorphismes d'un seul nucléotide (SNP), des haplotypes, des anomalies chromosomiques, des variations du nombre de copie (CNV), des modifications épigénétiques et des inversions d'ADN.

3. Méthode selon la revendication 1 ou la revendication 2, dans laquelle la variation génétique est :
un SNP présent sur le chromosome 1 qui est BIEC2-6883 ; et/ou
un SNP présent sur le chromosome 9 qui est BIEC2-1094991 ; et/ou
un SNP présent sur le chromosome 18 sélectionné parmi BIEC2-438205, BIEC2-416680, BIEC2-416681, BIEC2-438210, BIEC2-416683, BIEC2-438214, BIEC2-438222, BIEC2-438227, BIEC2-416704 ou BIEC2-416766.

4. Méthode selon l'une quelconque des revendications 1 à 3, dans laquelle la variation génétique est un SNP présent sur le chromosome 18 sélectionné parmi BIEC2-416680, BIEC2-416681 ou BIEC2-416704.

5. Méthode selon l'une quelconque des revendications 1 à 4, dans laquelle la variation génétique est un SNP présent sur le chromosome 18 qui est BIEC2-417495.

6. Méthode selon l'une quelconque des revendications 1 à 5, dans laquelle le sujet est un cheval et les variations génétiques sont dans l'un ou plusieurs des gènes suivants : ZNF804A, FSIP2, ITGAV, CALCRL, COL3A1, COL3A2, COL5A2 ou MSTN situés entre 61,7 Mb et 66,5 Mb sur le chromosome 18 du génome équin.

7. Méthode selon la revendication 6, dans laquelle les variations génétiques sont dans ZNF804A situé entre 61,7 Mb et 62,1 Mb sur le chromosome 18 du génome équin.

8. Méthode selon la revendication 6 ou la revendication 7, dans laquelle les variations génétiques dans ZNF804A sont sélectionnées parmi :
un ou plusieurs des SNP énumérés dans le tableau 1 ; et/ou
le SNP dans l'exon 4 de ZNF804A à la position de paire de bases 62045643 du chromosome 18 ; et/ou
le 3'-UTR du ZNF804A sélectionné parmi ceux énumérés dans le tableau 1 aux positions de paires de bases 62047178, 62047184, 62047262 et 62047293 du chromosome 18.

9. Méthode selon la revendication 6, dans laquelle les variations génétiques sont dans MSTN situé entre 61,7 Mb et 66,5 Mb sur le chromosome 18 du génome équin, par exemple à la position de paire de bases 66493737 du chromosome 18.

10. Méthode selon l'une quelconque des revendications 1 à 9, dans laquelle la variation génétique est :
un SNP présent sur le chromosome 21 qui est BIEC2-574084 ; et/ou
un SNP présent sur le chromosome 22 sélectionné parmi BIEC2-595969, BIEC2-596079, BIEC2-596530, BIEC2-596542 ou BIEC2-596546.

11. Méthode selon l'une quelconque des revendications 1 à 10, dans laquelle les variations génétiques sont dans ZFP64 situé entre 39,7 Mb et 39,9 Mb sur le chromosome 22 du génome équin.

12. Méthode selon la revendication 1, dans laquelle l'échantillon biologique est sélectionné parmi : du sang complet, du sérum sanguin, du plasma, de l'urine, de la salive ou un autre liquide corporel (selles, liquide lacrymal, liquide synovial, expectoration), des poils, du liquide céphalorachidien (LCR) ou d'un extrait ou d'une purification de ceux-ci, ou d'une dilution de ceux-ci, d'homogénats de tissus, de sections de tissus et de spécimens de biopsie.

13. Méthode selon l'une quelconque des revendications 1 à 12, dans laquelle l'étape de détection comprend une ou plusieurs des techniques suivantes : réaction en chaîne polymérase (PCR), réaction en chaîne polymérase - transcriptase inverse (RT-PCR), PCR nichée, réaction en chaîne ligase, amplification de signal d'ADN ramifié, reporteurs ARN amplifiables, réplication Q-bêta, amplification basée sur la transcription, amplification de l'ADN en boomerang, activation de déplacement de brin, technologie de cyclage par sonde ou amplification isothermique d'acide nucléique basée sur la séquence (NASBA), technologie Invader ou d'autres essais de réplication de séquence ou essais d'amplification de signal.

14. Procédé selon l'une quelconque des revendications 1 à 13, qui comprend de plus l'étape de calcul de la prédisposition au risque de fracture.
